# EUROPEAN PATENT SPECIFICATION

(11) **EP 1 440 311 B1**
(45) Date of publication and mention of the grant of the patent: **07.01.2009**
(21) Application number: 02757500.0
(22) Date of filing: 30.08.2002
(51) Int. Cl.: G01N 33/00, C07H 21/04

(54) **AFFINITY-SHIFTED PROBES FOR QUANTIFYING ANALYTE POLYNUCLEOTIDES**
SONDEN MIT AFFINITÄTSVERSCHIEBUNG ZUR QUANTIFIZIERUNG VON ANALYTENPOLYNUKLEOTIDEN
SONDES PRESENTANT DIFFERENTES AFFINITES POUR QUANTIFIER DES POLYNUCLEOTIDES D'UN ANALYTE

(30) Priority: 31.08.2001 US 316770 P; 26.03.2002 US 368072 P
(43) Date of publication of application: 28.07.2004
(73) Proprietor: GEN-PROBE INCORPORATED, San Diego, CA 92121-4362 (US)
(72) Inventor: BECKER, Michael, M., San Diego, CA 92131 (US); NELSON, Norman, C., San Diego, CA 92111 (US)
(74) Representative: Viering, Jentschura & Partner
(86) International application number: PCT/US2002/027710
(87) International publication number: WO 2003/020952

(56) References cited:
- WO-A-01/31062
- WO-A-99/23490
- WO-A-99/55912
- US-A- 4 766 062
- US-B1- 6 238 927
- VET J A M ET AL: "Multiplex detection of four pathogenic retroviruses using molecular beacons" PROCEEDINGS OF THE NATIONAL ACADEMY OF SCIENCES OF USA, NATIONAL ACADEMY OF SCIENCE. WASHINGTON, US, vol. 96, 1999, pages 6394-6399, XP002145609 ISSN: 0027-8424
- TYAGI ET AL.: 'Molecular beacons: probes that fluoresce upon hybridization' NATURE BIOTECHNOLOGY vol. 14, March 1996, pages 303 - 308, XP002949995
- YIP SHEA PING ET AL: "t Use of dual TaqMan probes to increase the sensitivity of 1-step quantitative reverse transcription-PCR: application to the detection of SARS coronavirus." CLINICAL CHEMISTRY, vol. 51, no. 10, October 2005 (2005-10), pages 1885-1888,
- LIVAK K.J.: "Allelic discrimination using fluorogenic probes and the 5' nuclease assay" GENETIC ANALYSIS: BIOMOLECULAR ENGINEERING, ELSEVIER SCIENCE PUBLISHING, vol. 14, 1999, pages 143-149, XP004158696
- LYAMICHEV V. ET AL: "Polymorphism identification and quantitative detection of genomic DNA by invasive cleavage of oligonucleotide probes" NATURE BIOTECHNOLOGY, vol. 17, March 1999 (1999-03), pages 292-296,

### SEQUENCE LISTING

<110> Becker, Michael M.
   Nelson, Norman C.
<120> Affinity-Shifted Probes for Quantifying
   Analyte Polynucleotides
<130> GP129-03.UT
<150> 60/316,770
   <151> 2001-08-31
<150> 60/368,072
   <151> 2002-03-26
<160> 16
<170> FastSEQ for Windows Version 3.0
<210> 1
   <211> 26
   <212> RNA
   <213> E. coli
<400> 1
   auguuggguu aagucccgca acgagc 26
<210> 2
   <211> 26
   <212> DNA
   <213> E. coli
<400> 2
   gctcgttgcg ggacttaacc caacat 26
<210> 3
   <211> 20
   <212> DNA
   <213> E. coli
<400> 3
   tgcgggactt aacccaacat 20
<210> 4
   <211> 21
   <212> RNA
   <213> Artificial Sequence
<220>
   <223> Molecular beacon specific for HIV-1
<221> misc_feature
   <222> (7)...(15)
   <223> Sequence complementary to HIV-1 target
<221> misc_feature
   <222> (1)...(21)
   <223> Construct created using 2'-methoxy nucleotide analogs
<400> 4
   ccgaguacag ugcaggcucg g 21
<210> 5
   <211> 22
   <212> RNA
   <213> Artificial Sequence
<220>
   <223> Molecular beacon specific for HIV-1
<221> misc_feature
   <222> (7)...(16)
   <223> Sequence complementary to HIV-1 target
<221> misc_feature
   <222> (1)...(22)
   <223> Construct created using 2'-methoxy nucleotide analogs
<400> 5
   ccgaguacag ugcagggcuc gg 22
<210> 6
   <211> 23
   <212> RNA
   <213> Artificial Sequence
<220>
   <223> Molecular beacon specific for HIV-1
<221> misc_feature
   <222> (7)...(17)
   <223> Sequence complementary to HIV-1 target
<221> misc_feature
   <222> (1)...(23)
   <223> Construct created using 2'-methoxy nucleotide analogs
<400> 6
   ccgaguacag ugcaggggcu cgg 23
<210> 7
   <211> 26
   <212> RNA
   <213> Artificial Sequence
<220>
   <223> Molecular beacon specific for HIV-1
<221> misc_feature
   <222> (7)...(20)
   <223> Sequence complementary to HIV-1 target
<221> misc_feature
   <222> (1)...(26)
   <223> Construct created using 2'-methoxy nucleotide analogs
<400> 7
   ccgaguggua cagugcaggg gcucgg 26
<210> 8
   <211> 28
   <212> RNA
   <213> Artificial Sequence
<220>
   <223> Molecular beacon specific for HIV-1
<221> misc_feature
   <222> (7)...(22)
   <223> Sequence complementary to HIV-1 target
<221> misc_feature
   <222> (1)...(28)
   <223> Construct created using 2'-methoxy nucleotide analogs
<400> 8
   ccgagugggg uacagugcag gggcucgg 28
<210> 9
   <211> 30
   <212> RNA
   <213> HIV-1
<400> 9
   uauucuuucc ccugcacugu accccccaau 30
<210> 10
   <211> 24
   <212> RNA
   <213> Artificial Sequence
<220>
   <223> Molecular beacon specific for HIV-1
<221> misc_feature
   <222> (1)...(24)
   <223> Construct created using 2'-methoxy nucleotide analogs
<400> 10
   ccgaggguac agugcagggc ucgg 24
<210> 11
   <211> 24
   <212> RNA
   <213> Artificial Sequence
<220>
   <223> Molecular beacon specific for HIV-1
<221> misc_feature
   <222> (1)...(24)
   <223> Construct created using 2'-methoxy nucleotide analogs
<400> 11
   gcguggguac agugcagggc acgc 24
<210> 12
   <211> 25
   <212> RNA
   <213> Artificial Sequence
<220>
   <223> Molecular beacon specific for HIV-1
<221> misc_feature
   <222> (1)...(25)
   <223> Construct created using 2'-methoxy nucleotide analogs
<400> 12
   ccgaggggua cagugcaggg uucgg 25
<210> 13
   <211> 26
   <212> RNA
   <213> Artificial Sequence
<220>
   <223> Molecular beacon specific for HIV-1
<221> misc_feature
   <222> (1)...(26)
   <223> Construct created using 2'-methoxy nucleotide analogs
<400> 13
   ccgaggggua cagugcaggg gcucgg 26
<210> 14
   <211> 23
   <212> RNA
   <213> Artificial Sequence
<220>
   <223> Molecular beacon specific for HIV-1
<221> misc_feature
   <222> (1)...(23)
   <223> Construct created using 2'-methoxy nucleotide analogs
<400> 14
   ccgaggguac agugcaggcu cgg 23
<210> 15
   <211> 25
   <212> RNA
   <213> Artificial Sequence
<220>
   <223> Molecular beacon specific for HIV-1
<221> misc_feature
   <222> (1)...(25)
   <223> Construct created using 2'-methoxy nucleotide analogs except for deoxy nucleotides at positions 5 and 21
<400> 15
   ccgaggggua cagugcaggg cucgg 25
<210> 16
   <211> 17
   <212> RNA
   <213> HIV-1
<400> 16
   gggguacagu gcagggg 17

### SEQUENCE LISTING

<110> Becker, Michael M.
   Nelson, Norman C.
<120> Affinity-Shifted Probes for Quantifying
   Analyte Polynucleotides
<130> GP129-03.UT
<150> 60/316,770
   <151> 2001-08-31
<150> 60/368,072
   <151> 2002-03-26
<160> 16
<170> FastSEQ for Windows Version 3.0
<210> 1
   <211> 26
   <212> RNA
   <213> E. coli
<400> 1
   auguuggguu aagucccgca acgagc 26
<210> 2
   <211> 26
   <212> DNA
   <213> E. coli
<400> 2
   gctcgttgcg ggacttaacc caacat 26
<210> 3
   <211> 20
   <212> DNA
   <213> E. coli
<400> 3
   tgcgggactt aacccaacat 20
<210> 4
   <211> 21
   <212> RNA
   <213> Artificial Sequence
<220>
   <223> Molecular beacon specific for HIV-1
<221> misc_feature
   <222> (7)...(15)
   <223> Sequence complementary to HIV-1 target
<221> misc_feature
   <222> (1)...(21)
   <223> Construct created using 2'-methoxy nucleotide analogs
<400> 4
   ccgaguacag ugcaggcucg g 21
<210> 5
   <211> 22
   <212> RNA
   <213> Artificial Sequence
<220>
   <223> Molecular beacon specific for HIV-1
<221> misc_feature
   <222> (7)...(16)
   <223> Sequence complementary to HIV-1 target
<221> misc_feature
   <222> (1)...(22)
   <223> Construct created using 2'-methoxy nucleotide analogs
<400> 5
   ccgaguacag ugcagggcuc gg 22
<210> 6
   <211> 23
   <212> RNA
   <213> Artificial Sequence
<220>
   <223> Molecular beacon specific for HIV-1
<221> misc_feature
   <222> (7)...(17)
   <223> Sequence complementary to HIV-1 target
<221> misc_feature
   <222> (1)...(23)
   <223> Construct created using 2'-methoxy nucleotide analogs
<400> 6
   ccgaguacag ugcaggggcu cgg 23
<210> 7
   <211> 26
   <212> RNA
   <213> Artificial Sequence
<220>
   <223> Molecular beacon specific for HIV-1
<221> misc_feature
   <222> (7)...(20)
   <223> Sequence complementary to HIV-1 target
<221> misc_feature
   <222> (1)...(26)
   <223> Construct created using 2'-methoxy nucleotide analogs
<400> 7
   ccgaguggua cagugcaggg gcucgg 26
<210> 8
   <211> 28
   <212> RNA
   <213> Artificial Sequence
<220>
   <223> Molecular beacon specific for HIV-1
<221> misc_feature
   <222> (7)...(22)
   <223> Sequence complementary to HIV-1 target
<221> misc_feature
   <222> (1)...(28)
   <223> Construct created using 2'-methoxy nucleotide analogs
<400> 8
   ccgagugggg uacagugcag gggcucgg 28
<210> 9
   <211> 30
   <212> RNA
   <213> HIV-1
<400> 9
   uauucuuucc ccugcacugu accccccaau 30
<210> 10
   <211> 24
   <212> RNA
   <213> Artificial Sequence
<220>
   <223> Molecular beacon specific for HIV-1
<221> misc_feature
   <222> (1)...(24)
   <223> Construct created using 2'-methoxy nucleotide analogs
<400> 10
   ccgaggguac agugcagggc ucgg 24
<210> 11
   <211> 24
   <212> RNA
   <213> Artificial Sequence
<220>
   <223> Molecular beacon specific for HIV-1
<221> misc_feature
   <222> (1)...(24)
   <223> Construct created using 2'-methoxy nucleotide analogs
<400> 11
   gcguggguac agugcagggc acgc 24
<210> 12
   <211> 25
   <212> RNA
   <213> Artificial Sequence
<220>
   <223> Molecular beacon specific for HIV-1
<221> misc_feature
   <222> (1)...(25)
   <223> Construct created using 2'-methoxy nucleotide analogs
<400> 12
   ccgaggggua cagugcaggg uucgg 25
<210> 13
   <211> 26
   <212> RNA
   <213> Artificial Sequence
<220>
   <223> Molecular beacon specific for HIV-1
<221> misc_feature
   <222> (1)...(26)
   <223> Construct created using 2'-methoxy nucleotide analogs
<400> 13
   ccgaggggua cagugcaggg gcucgg 26
<210> 14
   <211> 23
   <212> RNA
   <213> Artificial Sequence
<220>
   <223> Molecular beacon specific for HIV-1
<221> misc_feature
   <222> (1)...(23)
   <223> Construct created using 2'-methoxy nucleotide analogs
<400> 14
   ccgaggguac agugcaggcu cgg 23
<210> 15
   <211> 25
   <212> RNA
   <213> Artificial Sequence
<220>
   <223> Molecular beacon specific for HIV-1
<221> misc_feature
   <222> (1)...(25)
   <223> Construct created using 2'-methoxy nucleotide analogs except for deoxy nucleotides at positions 5 and 21
<400> 15
   ccgaggggua cagugcaggg cucgg 25
<210> 16
   <211> 17
   <212> RNA
   <213> HIV-1
<400> 16
   gggguacagu gcagggg 17

## Description

### Field of the Invention

The present invention relates to the field of analyte detection and quantitation. More specifically, the invention relates to the use of multiple probes for quantifying analytes over an extended dynamic range.

### Background of the Invention

The ability to amplify polynucleotide templates *in vitro* represents both an opportunity and a challenge. Early assays exploited the ability of amplification reactions to synthesize large amounts of amplicon, but were unable to relate starting amounts of a polynucleotide template with the amount of amplicon produced in the reaction. The fact that amplification reactions may be characterized by early periods of exponential amplification means that small changes in reaction efficiency may be reflected as disproportionately large changes in the amounts of specific product produced in the reaction. This confounds the relationship between the amount of input starting material and the amount of specific product synthesized when the efficiencies of two reactions differ.

Attention has recently turned to amplification assays that can be used for quantifying small numbers of polynucleotide templates. These techniques represent important tools for monitoring viral load in patients undergoing antiviral therapy, measuring the dynamics of cancer cell populations following chemotherapy, assessing the quality of public water sources, and assuring the safety of food products. Some quantitative amplification assays measure ratios of different polynucleotides in a population. Others measure absolute numbers of polynucleotide molecules.

The value of quantitative amplification assays was illustrated by the finding that plasma viral load, and not the number of CD4+ T cells, was the better predictor of progression to AIDS and death (Science272:1167 (1996)). Indeed, HIV-1 viral load testing, or the measurement of HIV-RNA blood levels, is increasingly being used to gauge the effectiveness of anti-retroviral drug therapy and monitor disease progression. This would not have been possible using assays that provided only qualitative results.

The ability to quantify small numbers of transcripts synthesized by particular cell types similarly benefits the monitoring of residual disease or tumor burden. For example, Johansson et al., in Clinical Chemistry 46:921 (2000) described methods of monitoring the number of melanoma cells circulating in blood by quantifying mRNA encoding an enzyme. The procedure involved amplification of an internal standard to generate calibration curves. Success of the method relied on the fact that tyrosinase is uniquely expressed in melanocytes, a cell type which is not normally present in blood. Results showed that quantitative monitoring of blood for circulating melanocytes using nucleic acid testing could detect changes in disease progression before clinical evidence of metastasis was evident. Max et al., in Melanoma Research 11:371 (2001) confirmed the importance of quantitative assays for detecting circulating melanocytes, and showed how real-time amplification could be used for quantifying tyrosinase transcripts.

It seems clear that different methods for quantifying pre-amplification amounts of analyte polynucleotide templates will be useful under different circumstances. Early semiquantitative approaches used for analyzing nucleic acid amplification reactions manipulated the number of input templates by employing limiting dilution techniques, and so did not lend themselves to high throughput assays. Methods involving manipulation of reaction conditions to obtain quantitative information have been disclosed in published International Patent Application WO 01/07661 and in U.S. Patent Nos. 5,705,365 and 5,710,029. Still other approaches for quantifying analyte polynucleotides following amplification employed external standards, or internal standards to better account for tube-to-tube variation in amplification efficiency. More recent procedures for determining the pre-amplification amount of an analyte polynucleotide are based on real-time monitoring of amplicon formation, sometimes using "molecular beacon" hybridization probes (see Tyagi et al., Nature Biotechnology 14:303 (1996)).

Molecular beacons are unitary, single-stranded oligonucleotide hybridization probes that can be used to report the presence of specific nucleic acids in solution. These probes have a "stem-and-loop" configuration and include an internally quenched fluorophore. The loop portion of a molecular beacon is a probe sequence complementary to a target polynucleotide. Arm sequences flanking the loop are complementary to each other, and so can anneal to form a helical stem structure. A fluorophore moiety is conventionally linked to the terminus of one arm, and a quencher moiety is conventionally linked to the terminus of the other. In the absence of bound target, the stem keeps these two moieties in close proximity so that the quencher extinguishes the fluorescence of the fluorophore. Upon encountering a target molecule, the loop portion of the molecular beacon forms a hybrid that is more stable than the stem. The molecular beacon then undergoes a spontaneous conformational reorganization that separates the arms of the stem, thereby separating the flurorphore and the quencher. As a result, a fluorescent signal can be emitted from the fluorophore in the absence of quenching. Since unhybridized molecular beacons do not fluoresce significantly, it is not necessary to remove them to detect the probe-binding event. U.S. Patent No. 6,103,476, the disclosure of which is incorporated by reference herein, fully describes how to make and use molecular beacon probes.

WO 99/23490 describes the use of multiple probes targeting non-overlapping regions of an analyte to extend the dynamic range of hybridization assays. The claimed method describes the use of multiple probes targeting regions of an analyte overlapping by at least one nucleotide to extend the dynamic range of hybridization assays.

Despite the availability of several different approaches for quantifying polynucleotides, there remains a need for simplified methods that can be used in conjunction with a variety of assay systems, including systems employing polynucleotide amplification. Further, there exists a need for techniques that minimize the opportunity for false-positive results arising from positive carry-over contamination of amplification products. Ideally, these technique should be adaptable to high throughput assays. The present invention addresses all of these needs.

### Summary of the Invention

A first aspect of the invention relates to a method for quantifying the amount of an analyte polynucleotide contained in a sample over a range extending from a lower limit amount to an upper limit amount. In accordance with this aspect of the invention, there is first a step for providing a probe reagent. The probe reagent includes a first probe that is complementary to a first analyte sequence which is contained within the analyte polynucleotide, this first probe having a first oligonucleotide sequence, and a second probe that is complementary to a second analyte sequence which is contained within the analyte polynucleotide, this second probe having a second oligonucleotide sequence. In accordance with this embodiment, the first analyte sequence and the second analyte sequence are contiguous with each other and share at least one nucleotide position in common. Additionally, the first probe hybridizes to the analyte polynucleotide with a first affinity and the second probe hybridizes to the analyte polynucleotide with a second affinity that is different from the first affinity.

There are numerous variations that apply to the reagent. For example, in accordance with one embodiment, the first probe and the second probe may be either soluble probes or immobilized probes. In either instance, the first oligonucleotide sequence may include the complement of the first analyte sequence, or alternatively may have exactly the complement of the first analyte sequence without additional sequence. In another instance, regardless of whether the two probes are soluble or immobilized, the first oligonucleotide sequence and the second oligonucleotide sequence may be identical to each other. When this is the case, at least one of the two probes may include a nucleotide analog. Again regardless of whether the two probes are soluble or immobilized, the first oligonucleotide sequence and the second oligonucleotide sequence may be different from each other. In this instance, the first and the second probes can each be self-reporting probes that include a detectable label. In one embodiment, the detectable labels of the two self-reporting probes may be identical detectable labels. Again regardless of whether the two probes are soluble or immobilized, the first probe may further include a first detectable label, and the second probe may further include a second detectable label. When this is the case, the two detectable labels may include either a chemiluminescent moiety or a fluorophore. When the two detectable labels are chemiluminescent moieties, the chemiluminescent moieties may be of identical types. In a particular case, the identical chemiluminescent moieties include acridinium ester. According to another embodiment, when the first probe and second probes are both soluble probes, they are soluble probes having a linear configuration. According to a different embodiment, when both of the probes are immobilized probes, they may be molecular beacon probes. According to a different embodiment, and again regardless of whether the two probes are soluble or immobilized, the first probe and the second probe may be first and second self-reporting probes. When this is the case, the first and second self-reporting probes may be first and second molecular beacons. In alternative embodiments of this invention, the first and second molecular beacons each include a stem portion and a loop portion, and either the first and second molecular beacons differ from each other in the length of their respective stem portions, or in the length of their respective loop portions. In still another embodiment, at least one of two molecular beacons includes at least one nucleotide analog.

Next, there is a step for hybridizing the probe reagent and any of the analyte polynucleotide that may be present in the sample. This is followed by a step for measuring a signal that indicates the magnitude of hybrid duplex formation in the hybridizing step, and is in turn followed by a step for quantifying from the measured signal the amount of analyte polynucleotide present in the sample. In accordance with different embodiments of the invention, either the first and second probes of the invented reagent are both soluble probes, or the first and second probes of the invented reagent are both immobilized probes. In another embodiment, the quantifying step involves comparing the measured hybridization signal to a standard curve. In yet another embodiment of the invention, the measuring step involves making an optical measurement. When this is the case, the measuring step may alternatively involve performing luminometry, or measuring by fluorometry.

Also described is a method of making a probe reagent for quantifying an analyte polynucleotide. According to this method, there is a step for selecting a first oligonucleotide probe that is complementary to a first analyte sequence which is contained within the analyte polynucleotide. This first oligonucleotide probe is capable of hybridizing to the analyte polynucleotide to form a first duplex having a first affinity. This is followed by a step for selecting a second oligonucleotide probe that is complementary to a second analyte sequence which also is contained within the analyte polynucleotide. This second oligonucleotide probe is capable of hybridizing to the analyte polynucleotide to form a second duplex having a second affinity. The first affinity and the second affinity are different from each other. One test for ensuring that different oligonucleotide probes hybridize to their respective analyte sequences with different affinities is to ensure that the two probe:target hybrids have different Tms when measured under identical buffer conditions and probe and target concentrations. In accordance with the invention, the first analyte sequence and the second analyte sequence are contiguous with each other and share at least one nucleotide position in common. After the probes are selected, they are next combined. In one embodiment, the first oligonucleotide probe comprises a first detectable label and the second oligonucleotide probe comprises a second label. In certain embodiments, the first and second detectable labels are identical. In a different embodiment of the invented method, there is an additional step for immobilizing the combined probes to a solid support.

### Definitions

The following terms have the following meanings for the purposes of this disclosure, unless expressly stated to the contrary herein.

As used herein, a "test sample" is a sample suspected of containing nucleic acids to be analyzed for the presence or amount of an analyte polynucleotide. Nucleic acids of the test sample may be of any biological origin, including any tissue or polynucleotide-containing material obtained from a human. For example, the nucleic acids of the test sample may be from a biological sample that may include one or more of: peripheral blood, plasma, serum, bone marrow, biopsy tissue including lymph nodes, respiratory tissue or exudates, gastrointestinal tissue, cervical swab samples, semen or other body fluids, tissues or materials. Biological samples may be treated to disrupt tissue or cell structure, thereby releasing intracellular components into a solution which may contain enzymes, buffers, salts, detergents and the like. Alternative sources of nucleic acids may include water or food samples that are to be tested for the presence of a particular analyte polynucleotide that would indicate the presence of a microorganism.

As used herein, an "oligonucleotide" or "oligomer" is a polymeric chain of at least two, generally between about five and about 100, chemical subunits, each subunit comprising a nucleotide base moiety, a sugar moiety, and a linking moiety that joins the subunits in a linear spacial configuration. Common nucleotide base moieties are guanine (G), adenine (A), cytosine (C), thymine (T) and uracil (U), although other rare or modified nucleotide bases able to hydrogen bond are well known to those skilled in the art. Oligonucleotides may be purified from naturally occurring sources, but preferably are synthesized using any of a variety of well known enzymatic or chemical methods and may contain nucleotide analogs. The term includes polymers containing analogs of naturally occurring nucleotides and particularly includes analogs having a methoxy group at the 2' position of the ribose (OMe).

As used herein, "polynucleotide" means either RNA or DNA, along with any synthetic nucleotide analogs or other molecules that may be present in the sequence and that do not prevent hybridization of the polynucleotide with a second molecule having a complementary sequence. The term includes polymers containing analogs of naturally occurring nucleotides and particularly includes analogs having a methoxy group at the 2' position of the ribose (OMe).

An "analyte polynucleotide" is a target polynucleotide that is to be detected, quantified or replicated by a nucleic acid amplification process, such as the below-described TMA protocol.

As used herein, a "detectable label" is a chemical species that can be detected or can lead to a detectable response. Detectable labels in accordance with the invention can be linked to probes either directly or indirectly. With particular reference to molecular beacons or other self-reporting probes, it is preferred for detectable labels to be members of an interactive label pair. It is highly preferred for one member of the label pair to be a fluorophore, and for the other member of the label pair to be a quencher. Examples of fluorophores and quenchers are given at column 5 in U.S. Patent No. 6,037,130.

"Homogeneous" assay formats employing hybridization probes do not require removal of unhybridized probe to determine accurately the extent of specific probe binding. In this way the synthesis of nucleic acids can be monitored as it is occurring, in sealed tubes, without interrupting the amplification reaction or performing additional manipulations.

A "homogeneous detectable label" refers to a label that can be detected in a homogeneous fashion by determining whether the label is on a probe hybridized to a target sequence. That is, homogeneous detectable labels can be detected without physically removing hybridized from unhybridized forms of the label or labeled probe.

As used herein, the "specific activity" of a detectably labeled probe is a measure of the abundance of the detectable label per unit of probe. For example, the specific activity of a polynucleotide probe harboring a chemiluminescent label may be indicated by a number of chemiluminescent light counts (Berthold Clinilumat) per picomole of probe.

As used herein, "amplification" or "nucleic acid amplification" or "polynucleotide amplification" refers to an *in vitro* procedure for obtaining multiple copies of a target nucleic acid sequence, its complement or fragments thereof.

An "amplicon" is a polynucleotide product generated in an amplification reaction.

An "analyte amplicon" is a polynucleotide product of an amplification reaction wherein an analyte polynucleotide served as the template for synthesis of polynucleotide copies or amplification products.

By "target" or "target polynucleotide" is meant a specific deoxyribonucleotide or ribonucleotide molecule containing a target nucleobase sequence which may be hybridized by a probe or amplification primer. Exemplary targets include viral polynucleotides, bacterial polynucleotides (such as rRNA), and eucaryotic mRNA. In the context of nucleic acid amplification reactions, a target polynucleotide includes a target sequence to be replicated, may be either single-stranded or double-stranded, and may include sequences in addition to the target sequence, which additional sequences may not be amplified.

A "target sequence" refers to the particular nucleotide sequence of the target polynucleotide which may be hybridized by a complementary detection probe or amplification primer.

As used herein, two sequences that are contained within a single polynucleotide are said to be "contiguous" with each other if there are no intervening nucleotides between the two sequences. Two sequences that are both contained within a larger sequence and that are contiguous with each other are said to "share nucleotide positions in common" only when there is overlap between the two polynucleotide sequences.

As used herein, contiguous sequences can be "overlapping" . Examples of two overlapping contiguous sequences would be two contiguous sequences that share in common at least 1 nucleotide position, at least 3 nucleotide positions, at least 5 nucleotide positions, at least 10 nucleotide positions, or all of the nucleotides that span their entire lengths. An example of two contiguous sequences that are non-overlapping would be two sequences that abut each other end-to-end, as may result from cleavage of the backbone of a single-stranded polynucleotide.

As used herein, a probe includes a sequence complementary to an "analyte sequence" which is contained within an analyte polynucleotide. It should be clear that "analyte sequence" refers only to the portion of the analyte polynucleotide that participates in hybrid duplex formation by base pairing. The sequence of the probe may have a length and sequence exactly complementary to the analyte sequence, in which case the probe sequence is said to "consist" of the complement of the analyte sequence. Alternatively, the probe may include additional sequence which does not find its complement in the analyte polynucleotide. This additional sequence may, for example, participate in secondary structure formation. A molecular beacon that includes a pair of "arm" sequences complementary to each other, but not complementary to the sequence of the analyte polynucleotide illustrates this latter option. In such a case, the probe sequence is said to "comprise" the complement of the analyte sequence.

By "transcription associated amplification" is meant any type of nucleic acid amplification that uses an RNA polymerase to produce multiple RNA transcripts from a nucleic acid template. One example of a transcription associated amplification method, called "Transcription Mediated Amplification" (TMA), generally employs an RNA polymerase, a reverse transcriptase, deoxyribonucleoside triphosphates, ribonucleoside triphosphates, and a promoter-template complementary oligonucleotide, and optionally may include one or more analogous oligonucleotides. Variations of TMA are well known in the art as disclosed in detail in Burg et al., U.S. Patent No. 5,437,990; Kacian et al., U.S. Patent Nos. 5,399,491 and 5,554,516; Kacian et al., PCT No. WO 93/22461; Gingeras et al., PCT No. WO 88/01302; Gingeras et al., PCT No. WO 88/10315; Malek et al., U.S. Patent No. 5,130,238; Urdea et al., U.S. Patent Nos. 4,868,105 and 5,124,246; McDonough et al., PCT No. WO 94/03472; and Ryder et al., PCT No. WO 95/03430. The methods of Kacian et al., are preferred for conducting nucleic acid amplification procedures of the type disclosed herein.

By "dynamic range" is meant a linear or predictably accurate correspondence between the level of analyte present in the sample to be assayed and the amount of signal obtained from the label used to indicate the analyte's presence.

As used herein, an "array" is an orderly spatial arrangement of samples. It provides a medium for matching known and unknown nucleic acid samples based on base-pairing rules, and for automating the process of identifying and/or quantifying unknowns. Although arrays that are useful in connection with the invention may have any number of spatially separated samples or "spots" contained therein, certain preferred arrays have 1-100 spots, more preferably 2-64 spots, more preferably 3-12 spots, and still more preferably 3-9 spots.

As used herein, a "molecular beacon" or "molecular beacon probe" is a nucleobase probe having a stem-and-loop structure that hybridizes specifically to a target polynucleotide under conditions that promote hybridization to form a detectable hybrid. Molecular beacons have been described in U.S. Patent No. 6,103,476.

With reference to molecular beacons or other probes, "immobilized" is meant to convey that the probe joins, directly or indirectly, to a solid support. Immobilized probes may be joined to the solid support by covalent or non-covalent interactions.

As used herein, a "measurable binding interaction" is an interaction between a target sequence and a probe that yields a detectable signal.

By "binding affinity" is meant a measure of the strength of the interaction between two binding partners. When the binding partners are nucleic acids that are at least partly complementary, the binding affinity is a measure of the strength of hydrogen bonding under defined nucleic acid hybridization conditions. A convenient measure of nucleic acid binding affinity is the Tm, which is the temperature at which 50% of said two strands are in the double-stranded or hybridized form.

When a modified oligonucleotide is referred to as having an "increased" or "greater" affinity or rate, it is meant that the rate of hybridization or the affinity of the modified oligonucleotide is greater than the hybridization rate or binding affinity of an unmodified oligonucleotide of the same length and base sequence to the same target.

As used herein, a "quenching ratio" is a corrected signal-to-noise ratio that is calculated by first subtracting the signal measured for a negative control reaction (such as a buffer control) from the signal measured for a sample that included a molecular beacon and a target polynucleotide, and then dividing that result by the value obtained by subtracting the signal measured for a negative control reaction from the signal measured for a sample containing the molecular beacon alone.

As used herein, the term "hybridization profile" refers to a relationship between the extent of probe hybridization and the amount of target polynucleotide included in a hybridization reaction. The hybridization profile is conveniently expressed graphically on a plot having the target amount on the x-axis and the hybridization signal on the y-axis. The resulting curve has a characteristic sigmoid shape.

As used herein, an "affinity-shifted probe reagent" includes at least two probes that are able to hybridize an analyte polynucleotide species with different affinities. The probes may be soluble or immobilized probes. A quantitative probe array is a construct that includes immobilized affinity-shifted probes.

As used herein, an "extended dynamic range assay" using an affinity-shifted probe reagent that comprises more than one probe in an assay wherein the affinity-shifted probe reagent allows detection and quantitation of an analyte over a range of analyte amounts in a sample that is greater than an amount of the analyte that can be detected by any one of the labeled probes present in the affinity-shifted probe reagent.

### Brief Description of the Drawings

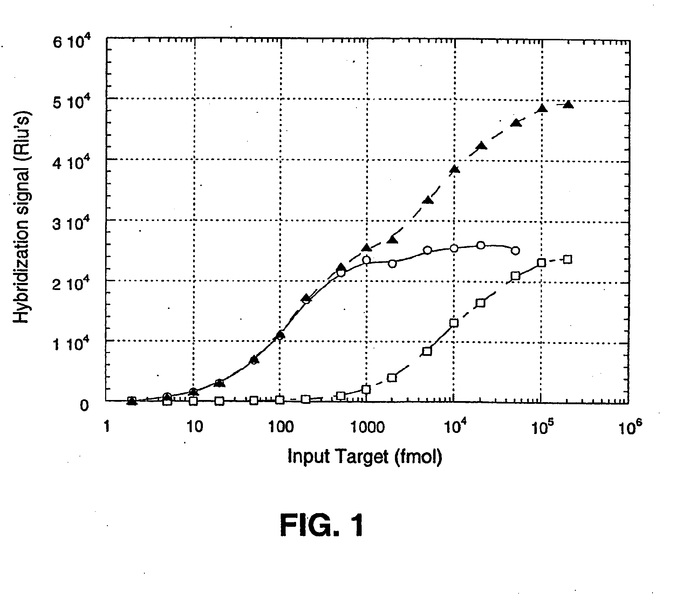

Figure 1 is a graph showing sigmoid curves representing the hybridization profiles of 26-mer (○) and 20-mer (□) probes. Also shown is a composite curve (▲) representing the additive effects resulting from hybridization of the two probes used in combination.

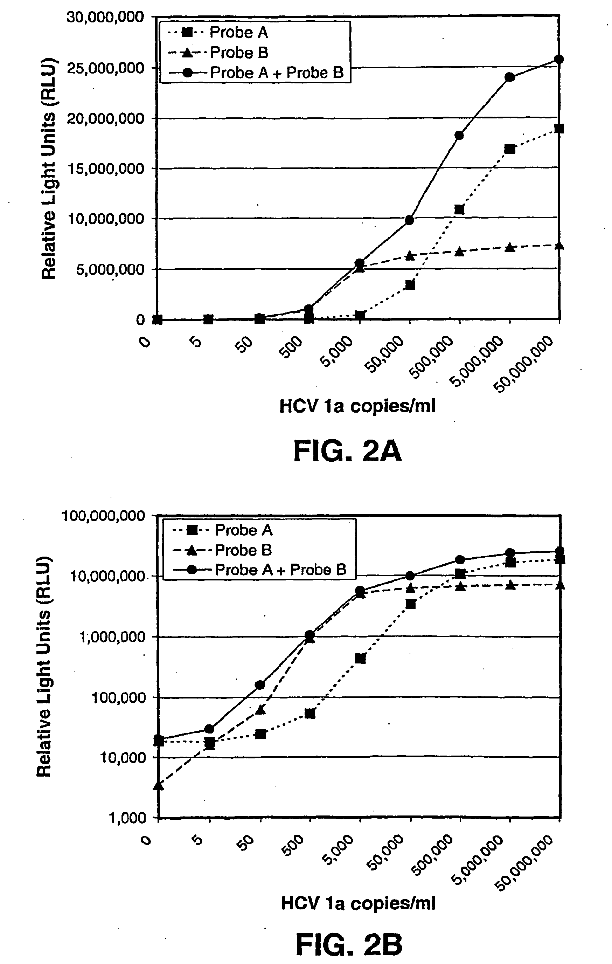

Figures 2A-2B are graphs showing hybridization signal strength as a function of increasing amounts of template polynucleotide input into three TMA reactions. Probe A (■) is a DNA probe, while Probe B (▲) includes 2'-OMe analogs and so exhibits higher affinity for analyte amplicons. Also shown is a composite curve (●) representing the additive effects from hybridizing Probe A in combination with Probe B. Both of the probes hybridize identical sequences within the analyte amplicon. The data plotted in Fig. 2A is re-plotted in Fig. 2B using a log-log scale.

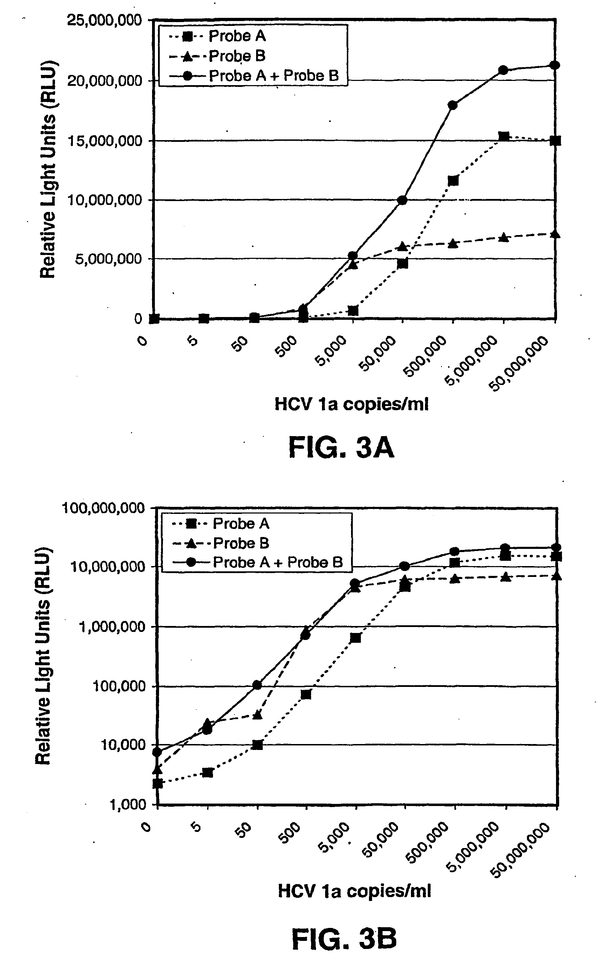

Figures 3A-3B are graphs showing hybridization signal strength as a function of increasing amounts of template polynucleotide input into three TMA reactions. Probe A (■) is a DNA probe, while Probe B (▲) includes 2'-OMe analogs and so exhibits higher affinity for analyte amplicons. Also shown is a composite curve (●) representing the additive effects from hybridizing Probe A in combination with Probe B. Both of the probes hybridize identical sequences within the analyte amplicon. The data plotted in Fig. 3A is re-plotted in Fig. 3B using a log-log scale.

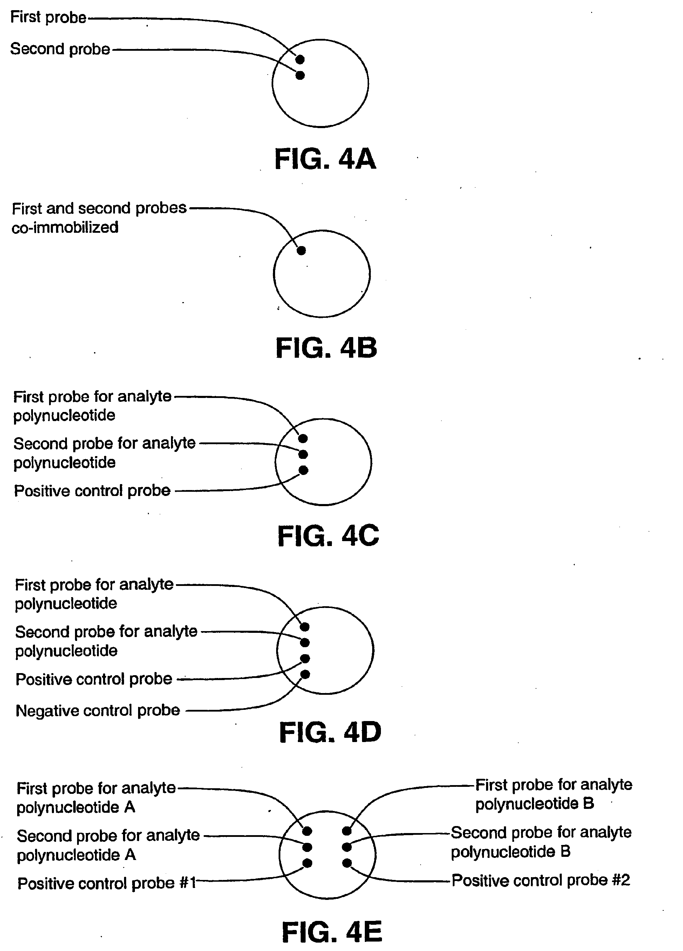

Figures 4A-4E show a series of schematic representations of arrayed microtiter wells.

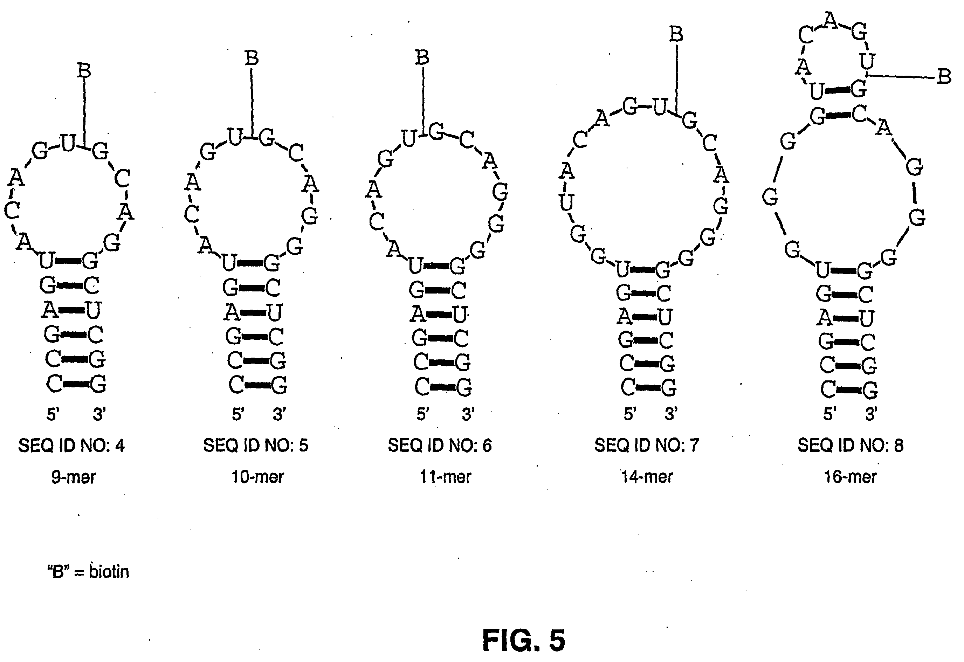

Figure 5 diagrammatically illustrates a collection of molecular beacon hybridization probes in their closed conformations. *Fluorophore* and quencher moieties are omitted from the illustration.

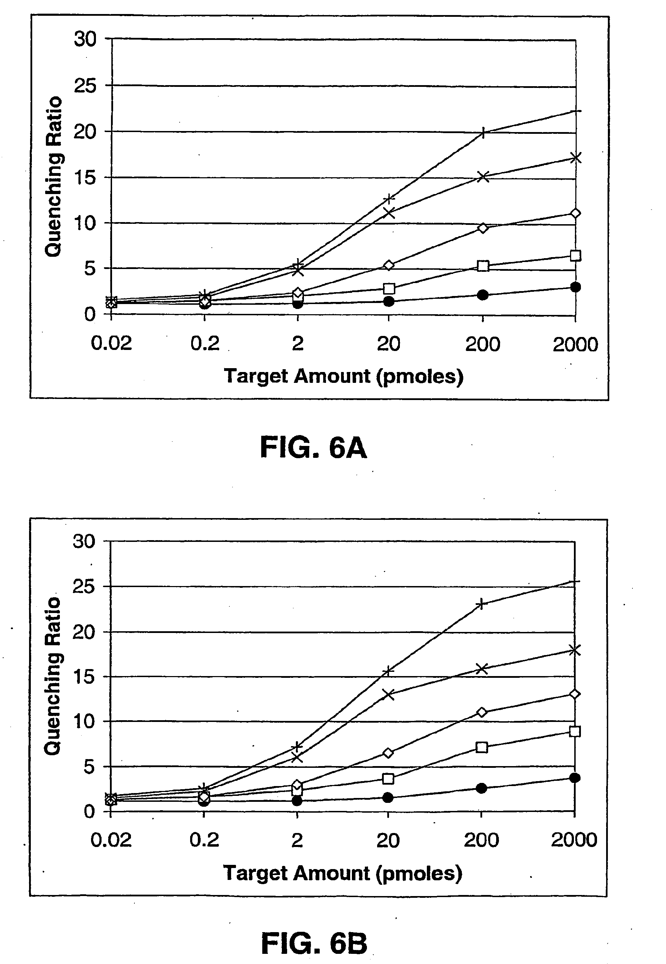

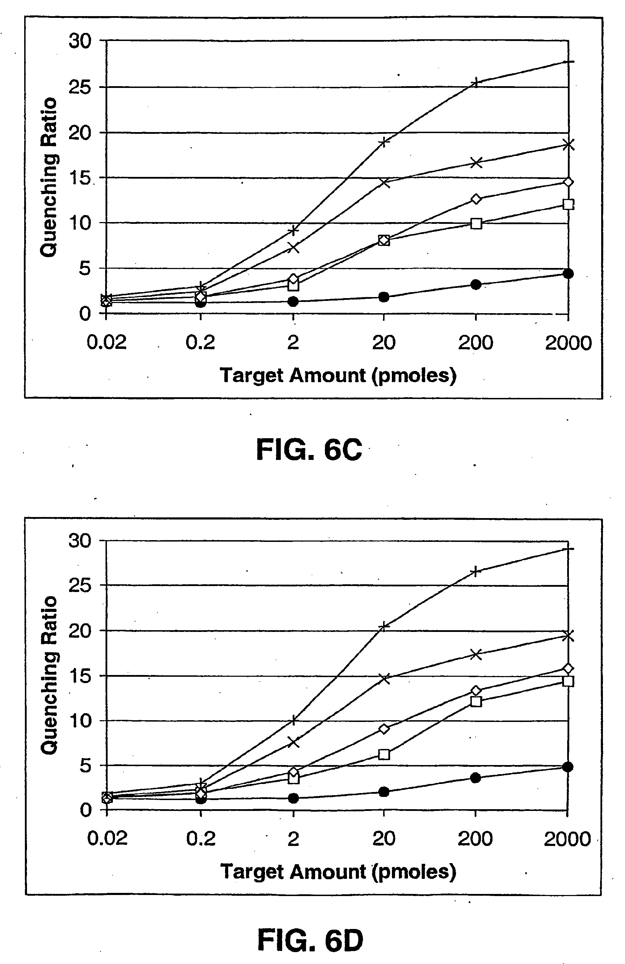

Figures 6A-6E are a collection of line graphs showing hybridization results for a series of five molecular beacon probes across a range of polynucleotide target concentrations after 1 hour (Fig. 6A), 2 hours (Fig. 6B), 3 hours (Fig. 6C), 5 hours (Fig. 6D) and 7 hours (Fg. 6E). Probes used in the procedure were a 9-mer (●), 10-mer (D), 11-mer (◊), 14-mer (+), and 16-mer (×).

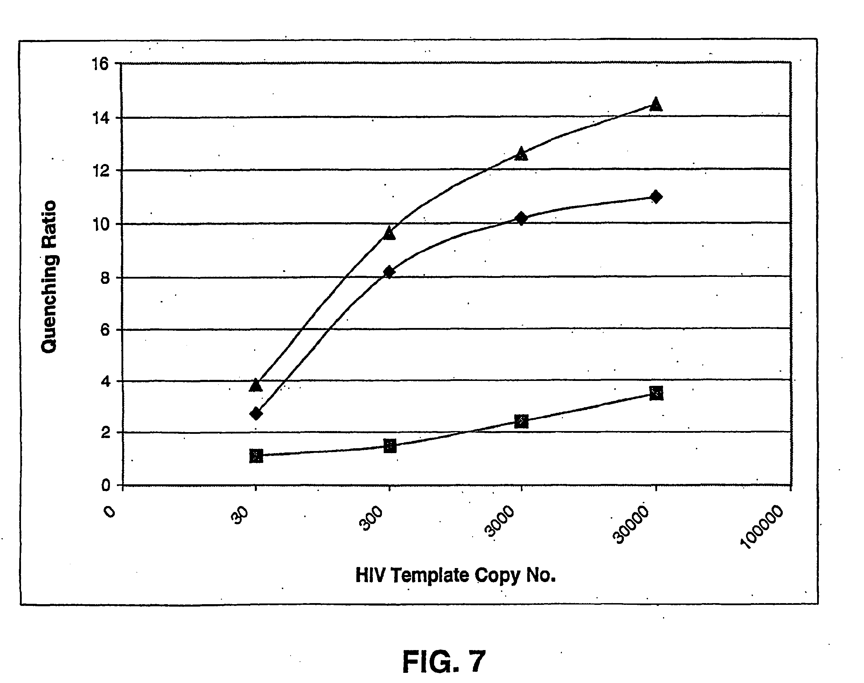

Figure 7 is a graph showing hybridization results (Quenching Ratios) for polynucleotide amplification reactions conducted in contact with quantitative probe arrays as a function of the number of copies of an HIV analyte polynucleotide template using two different molecular beacon probes. Curves shown in the graph represent results from the WT016 (◆) and WT013 (■) molecular beacons. Also shown on the graph is a composite curve (▲) that results from adding the hybridization signals of the individual probes.

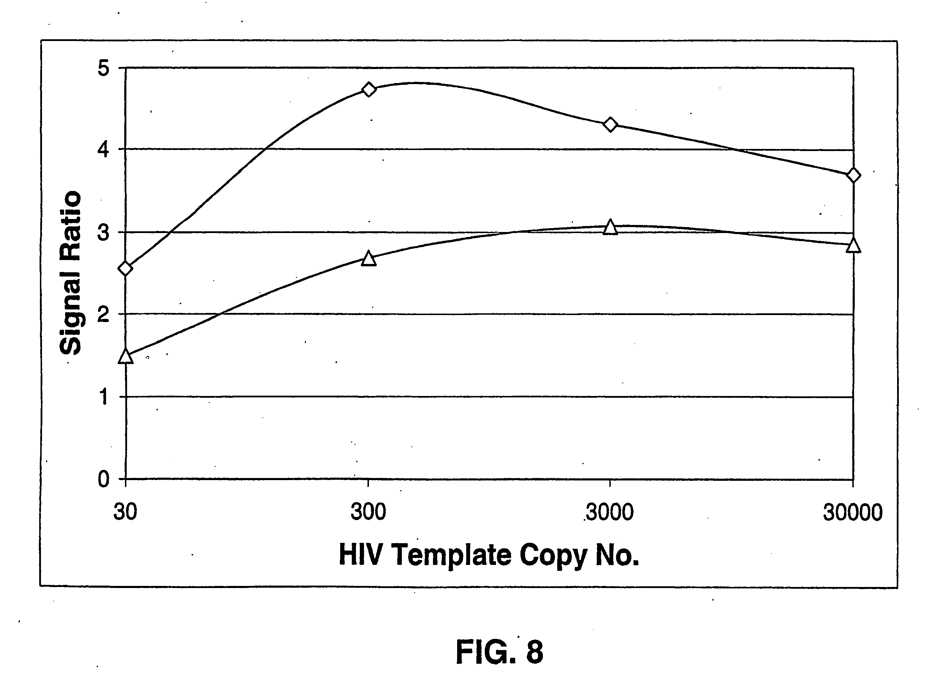

Figure 8 is a graph showing the signal ratios for two different combinations of molecular beacon probes as a function of the number of copies of an HIV analyte polynucleotide template. Curves shown in the graph represent the ratio of the WT016/WT013 (◊), and WT015dCG/WT013 (△) probes.

### Detailed Description of the Invention

The present invention provides methods for quantifying analyte polynucleotides in a format appropriate for the needs of clinical testing laboratories. The invention incorporates a feature whereby the dynamic range of the quantitative assay, meaning the range of analyte concentrations or amounts which reliably can be measured in the assay, is advantageously improved or "extended." The invention can be practiced using either soluble probes or immobilized probes. In one embodiment, the probes are immobilized in an array or microarray format to produce what are referred to herein as "quantitative probe arrays."

The extended dynamic range aspect of the invention stems from our discovery that a plurality of probes, each having different measurable binding interactions with a single analyte polynucleotide, can be used for quantifying the analyte over a broad range of concentrations or amounts. As disclosed herein, a plurality of polynucleotide probes labeled with either chemiluminescent moieties or fluorophore/quencher pairs have been used for quantifying polynucleotide analytes using either soluble or immobilized probe formats.

In highly preferred embodiments of the invention, different sets of two labeled probes in soluble form were used to quantify either known amounts of a target polynucleotide, or the products of a nucleic acid amplification reaction that was performed using known amounts of template polynucleotide. In both instances, the amounts of each of two nucleic acid probes used for quantifying the target polynucleotides were substantially lower than the upper limits of the analyte polynucleotide amounts that could be quantified. For example, as disclosed below in Example 1, 0.5 fmols of a soluble 26-mer probe was shown to be useful for measuring quantities of target analyte polynucleotides as high as 1,000 fmols. Similarly, 0.5 fmols of a soluble 20-mer probe having a sequence contained entirely within the 26-mer sequence was useful for measuring quantities of target analyte polynucleotides as high as 100,000 fmols, an amount that was 200,000 fold greater than the amount of probe used in the assay. In accordance with the invention, the dynamic range of a hybridization assay can be extended by the use of affinity-shifted probe reagents to an upper limit that is higher than the amount of any of the probes used in the assay.

In another highly preferred embodiment of the invention, a plurality of immobilized probes, more preferably immobilized self-reporting probes, still more preferably immobilized molecular beacon probes can be used for creating quantitative probe arrays having an enhanced capacity for quantifying analyte polynucleotides over a broad range of concentrations or amounts. For example, two molecular beacons having different affinities for the same or a different target region of a single analyte polynucleotide can be used for this purpose. As indicated below, analyte polynucleotides were quantified directly when a test sample contains sufficiently high numbers of these molecules to generate a detectable signal. However, both soluble probe formats and quantitative probe arrays were also useful for quantifying very low numbers of analyte polynucleotides when used in conjunction with nucleic acid amplification reactions.

Indeed, the present invention advantageously combines the features of nucleic acid amplification with a system for quantifying analyte polynucleotides in a format appropriate for clinical diagnostic testing. To avoid possible carry-over contamination from positively amplifying samples, it was a particular objective to eliminate or make optional any steps involving the physical transfer of post-amplification reaction mixtures between different containers in order to carry out detection and/or quantitation steps. While it initially seemed desirable to include hybridization probes in amplification reactions, thereby allowing amplification and detection/quantitation procedures to be combined, we discovered that some amplification reactions could be inhibited by the presence of hybridization probes. More specifically, we discovered that isothermal amplification reactions which generate amplicons complementary to the hybridization probe sometimes could be inhibited by the presence of hybridization probes. Accordingly, it was a further goal of the present invention to minimize the effects of hybridization probes that are included in isothermal amplification reactions.

As stated above, isothermal amplification reactions conducted in the presence of hybridization probes sometimes exhibited reduced amplification efficiency. While not wishing to be bound by any particular theory, it is possible that regions of duplex structure which characterize a probe:target complex may inhibit synthetic activity of polymerase enzymes if the target polynucleotide also serves as a template in the amplification reaction. Since amplification reactions based on thermal cycling procedures naturally employ high temperature steps that separate a hybridization probe from its target, the presence of probes in thermal cycling reaction mixtures would not be expected to have the same detrimental effects that were observed in isothermal reactions. Although created in response to requirements which may uniquely characterize isothermal amplification systems, the present invention can also be used in conjunction with amplification reactions based on thermal cycling. Indeed, the present invention is generally useful for quantifying analyte polynucleotides using any number of different amplification protocols.

The quantitative probe arrays disclosed herein advantageously can employ only a tiny fraction of the amount of probe that would otherwise be used in more conventional quantitative methods. Where picomolar amounts of a molecular beacon probe may have been used previously to quantify the analyte polynucleotide in solution, the approach described herein advantageously may employ 10,000-100,000 fold less probe without sacrificing the integrity of the results. This ability to achieve good quantitative results using lower amounts of hybridization probe actually allows isothermal amplification reactions to be conducted in contact with the hybridization probes of a quantitative probe array. As a result, amplification, detection and/or quantitative procedures can be conducted in an entirely closed format. This minimizes the possibility of carry-over contamination of negative test samples by amplicons produced in positively amplifying reactions.

To best exploit the potential of any quantitative technique, it is desirable to detect differences in the amount of an analyte polynucleotide over a broad range of concentrations or amounts. This is commonly referred to in the laboratory arts as a broad "dynamic range." Thus, another advantage of the present invention is the ability to extend the dynamic range of a single hybridization assay, thereby enhancing the capacity for quantifying an analyte polynucleotide over a broad range of several orders of magnitude. Alternative methods of extending the dynamic ranges of quantitative polynucleotide hybridization assays have been described previously.

For example, Nelson in U.S. Patent No. 6,180,340 describes a method which differs from the present invention in several important respects. More specifically, the present invention can employ identical, indistinguishable labels for labeling a plurality of probes to be used for quantifying analyte polynucleotides, even when specific activities of the labeled probes are not inversely related to the amounts of the probes used in the assay, and when the amount of probe in the assay is less than the amount of target polynucleotide that is to be quantified. The present invention does not require that different probes harboring identical labels must have different specific activities, or even be distinguishable by any other means. Indeed, in accordance with certain embodiments of the present invention two different probes bearing indistinguishable labels may be combined in a single hybridization reaction using soluble probes, or at a single spot within an array and give good quantitative results. In contrast to the methods described by Nelson, polynucleotide quantitation can be carried out in accordance with the present invention using a plurality of different probes that bind overlapping sequences, or even identical sequences within a single analyte polynucleotide. Competition among different probes for hybridization with a single target region has no substantial negative effect on the quantitative capacity of the invention. In yet another different respect, the present invention generally provides quantitative information about an analyte polynucleotide even when the amount of probe employed in the hybridization procedure is far below the amount or concentration of analyte polynucleotide that is to be detected and quantified by that probe in an assay.

In any assay for quantifying analyte polynucleotides using a hybridization protocol, it is desirable for the magnitude of the hybridization signal to fall within a substantially linear portion of a sigmoid standard control curve. Those having an ordinary level of skill in the art appreciate that a standard curve relates the amount of polynucleotide on a first axis and hybridization signal strength on a second axis. When the magnitude of the hybridization signal falls within non-linear portions of the standard control curve, or within portions of the standard control curve wherein hybridization signal strength has substantially plateaued with respect to increasing amounts of target polynucleotide, small levels of uncertainty in hybridization signal strength can undesirably exaggerate or otherwise introduce uncertainty about the amount of analyte polynucleotide present in a sample. To facilitate accurate quantitative determinations, it is highly desirable to have available one or more standard control curves characterized by extended regions which increase in a substantially linear fashion.

### Basis for Extending the Dynamic Range of Polynucleotide Hybridization Assays

The dynamic range of an assay conducted using either soluble or immobilized probes is extended through the use of a plurality of hybridization probes, each of the different probes having a different measurable interaction with the same analyte polynucleotide. In the context of the invention, a different measurable interaction is any feature that leads two different probes to have different hybridization profiles when hybridized with an analyte polynucleotide standard over a range of analyte amounts or concentrations. These hybridization profiles have a characteristic sigmoid shape that exhibits saturation at high levels of analyte polynucleotide.

Sets of probes having different measurable interactions with a particular target polynucleotide can result from any number of structural differences between the probes. For example, two probes can have overlapping or non-overlapping sequences and/or can differ by virtue of substitution of nucleotide analogs for conventional deoxyribonucleotides, even in probe sequences that are otherwise the same. If the probes are molecular beacons, then sets of molecular beacons can have identical target-complementary loop sequences, but differ in the sequences or lengths of their stem regions. Sets of molecular beacons can have identical stem regions, but differ in the sequence, length or secondary structure of their target-complementary loop sequences.

The concept underlying the extended dynamic range feature of the invention is illustrated by the following example. If two probes hybridize a single species of analyte polynucleotide with differing affinities, then the higher affinity probe will hybridize the analyte polynucleotide in preference to the lower affinity probe at any given level of analyte polynucleotide. As a result, each probe interacts with the analyte polynucleotide over a range of concentrations to produce a different sigmoid curve that relates the amount or concentration of analyte polynucleotide on a first axis and a measure of the extent of probe hybridization on a second axis. Importantly, as a result of the different hybridization characteristics of the two probes, the sigmoid curves representing hybridization of the individual probes will be non-overlapping and will be shifted with respect to each other on the first axis of the plot. When the two curves representing the responses of the different hybridization probes are combined on the second axis, for example by a simple additive process, the result will be a composite curve advantageously characterized by an extended dynamic range when compared with either of the individual curves. This is true when a plurality of sigmoid curves representing the responses of individuals among a plurality of hybridization probes are combined. More particularly, the resulting composite curve will be characterized by an extended dynamic range when compared with any of the constituent curves.

When different probes carry identical detectable labels, the hybridization signals from the probes cannot be distinguished. If a test sample containing an amount of an analyte polynucleotide is hybridized with two different hybridization probes, each having a different affinity for the analyte and bearing identical detectable labels, or detectable labels that are not distinguished during a detection step, then the measured hybridization signal will be a composite of the signals resulting from the two different probes. Thus, in certain preferred embodiments of the invention, the multiple probes contained in an affinity-shifted probe reagent, including quantitative probe arrays, have detectable labels that are not distinguished from each other during a step for detecting hybridization signal strength. It is not even necessary to distinguish hybridization signals attributed to the different probes to achieve success when using the invented method.

These concepts are illustrated by the experimental results presented in Figure 1. This figure shows a graph wherein the sigmoid plots representing the hybridization profile of a high affinity probe (i.e., the 26-mer) and a low affinity probe (i.e., the 20-mer) essentially combined to yield a composite curve when the hybridization reaction contained equimolar amounts of the two probes. The composite curve was characterized by an extended dynamic range with respect to either of the isolated constituent curves representing results obtained using the probes individually. The benefits of an extended dynamic range can be achieved even when the different probes are not used in the same hybridization reaction, and even when they are not mixed together or disposed in an array at a single locus.

Significantly, absolute amounts of an analyte polynucleotide can be determined using either soluble or immobilized probes, including probes immobilized in quantitative probe arrays, under conditions of target excess rather than conditions of probe excess. Indeed, the quantitative procedure can be carried out using exceedingly small amounts of each of the different hybridization probes in an array format.

As illustrated in the working Examples presented below, extended dynamic range hybridization assays can be performed using either soluble or immobilized probes. When used in connection with nucleic acid amplification protocols, assays employing either of these probe types can be conducted at the conclusion of the amplification reaction (i.e., an endpoint assay), or in a time-dependent manner as the amplification reaction progresses. This latter case may involve periodically monitoring the magnitude of probe hybridization during an isothermal amplification reaction, or between the cycles of a PCR or other thermal cycling amplification procedure.

Example 1 illustrates the general features of an extended dynamic range hybridization assay that employs soluble probes. Example 2 provides another illustration of the assay wherein soluble probes were hybridized to the products of a nucleic acid amplification reaction according to procedures substantially similar to those described in Example 1. Subsequent Examples presented herein describe the use of immobilized probes for quantifying analyte polynucleotides over an extended dynamic range. Indeed, the invention may be practiced using probes immobilized in array or microarray formats. Molecular beacons are highly preferred examples of probes that may be used in either soluble or immobilized probe formats. Each of these embodiments of the invention may be practiced using high-throughput assay formats.

When combinations of two or more soluble probes are used for quantifying analyte polynucleotides, the probes preferably are combined and used together in a single hybridization reaction to simplify the hybridization and detection steps. This is especially desirable when the amount of material to be quantified is limiting or when dividing a liquid sample into multiple aliquots is impractical or contraindicated. For example, it is often beneficial to avoid physical transfer of post-amplification reaction mixtures to minimize the risk of carryover contamination. Thus, two or more probes that hybridize the same target polynucleotide with differing affinities can be combined, simultaneously added to a post-amplification reaction mixture, hybridized with target amplicons contained in that mixture, and the magnitude of probe hybridization detected subsequently. The procedures underlying this embodiment of the invention are illustrated below in Examples 1 and 2.

The advantage of an extended dynamic range that results from the use of a plurality of different hybridization probes may be extended to immobilized probe and array formats. Since it is not necessary to use conditions wherein substantially all analyte polynucleotides in the sample are hybridized by probe, the amount of probe can be reduced dramatically when compared with prior art methods. The different hybridization probes can be combined at a single locus or "spot" in an array format, or alternatively may be immobilized at distinct loci. In certain preferred embodiments, the plurality of different hybridization probes for quantifying an analyte are combined at a single spot in an array. In other preferred embodiments, the plurality of hybridization probes for quantifying an analyte are disposed at different spots within an array. Indeed, there are advantages to immobilizing different probes that hybridize the same analyte polynucleotide at different loci within an array.

For example, when the hybridization signal from one probe is in a linear range of a sigmoid plot of the hybridization signal strength versus input target amount, but the signal for a different probe is in a non-linear range of an equivalent plot, it may be desirable to employ different probes at distinguishable loci. When an analyte polynucleotide is present in a test sample in an amount sufficient to saturate the signal-generating capacity of a high affinity probe, yet appropriate to fall within the substantially linear portion of the standard curve for a low affinity probe spotted at the same locus within the array, the signal from the high affinity probe effectively increases the background signal of the composite curve in the region that reflects the contribution of the low affinity probe. Stated differently, it may be desirable to detect hybridization signals particularly falling within the linear portions of one or more sigmoid curves without reading the signal from another probe that yields a saturated, non-linear response. This can be accomplished by reading individual signals from distinct hybridization probes that harbor distinguishable labels or that are immobilized at different spots in an array.

### General Features of Probes that may be Used in Quantitative Probe Arrays

Preferred detectable labels for probes in accordance with the present invention are detectable in homogeneous assay systems. Examples of these labels include chemiluminescent moieties, fluorescent moieties, luminescent moieties, and redox-active moieties that are amenable to electronic detection methods. Particularly preferred probes for use in quantitative probe arrays are self-reporting hybridization probes.

As indicated above, one example of a self-reporting hybridization assay probe is a structure commonly referred to as a "molecular beacon." These probes comprise nucleic acid molecules having a target complement sequence, an affinity pair (or nucleic acid "arms") holding the probe in a closed conformation in the absence of a target nucleic acid sequence, and a label pair that interacts when the probe is in a closed conformation. Hybridization of the target nucleic acid and the target complement sequence separates the members of the affinity pair, thereby shifting the probe to an open confirmation. The shift to the open confirmation is detectable due to reduced interaction of the label pair, which may be, for example, a fluorophore (e.g., fluorescein and Cy5) and a quencher (e.g., DABCYL and EDANS). Molecular beacons are fully described in U.S. Patent No. 5,925,517 A second type of a self-reporting probe that may be used in connection with the present invention also exhibits some degree of self-complementarity. More particularly, structures referred to as "molecular torches" are designed to include distinct regions of self-complementarity (coined "the target binding domain" and "the target closing domain") which are connected by a joining region and which hybridize to one another under predetermined hybridization assay conditions. When exposed to denaturing conditions, the two complementary regions (which may be fully or partially complementary) of the molecular torch melt, leaving the target binding domain available for hybridization to a target sequence when the predetermined hybridization assay conditions are restored. Molecular torches are designed so that the target binding domain favors hybridization to the target sequence over the target closing domain. The target binding domain and the target closing domain of a molecular torch include interacting labels (e.g., fluorescent/quencher) positioned so that a different signal is produced when the molecular torch is self-hybridized as opposed to when the molecular torch is hybridized to a target nucleic acid, thereby permitting detection of probe:target duplexes in a test sample in the presence of unhybridized probe having a viable label associated therewith. Molecular torches are fully described in U.S. Patent No. 6,361,945.

Preferred electronic labeling and detection approaches are disclosed in U.S. Patent Nos. 5,591,578 and 5,770,369, and the published international patent application WO 98/57158. Redox active moieties useful as labels in the present invention include transition metals such as Cd, Mg, Cu, Co, Pd, Zn, Fe and Ru.

### General Features of Soluble Probes Used in Extended Dynamic Range Assays

A wide variety of detectable labels may be used with success in embodiments of the invention that employ labeled forms of soluble probes. For example, the multiple probes used for carrying out the present invention may be labeled with radioisotopes, chemiluminescent moieties, fluorescent moieties, enzymes, haptens, or even unique oligonucleotide sequences. Other highly preferred detectable labels used for labeling soluble probes employed in extended dynamic range hybridization assays include homogeneous detectable labels that can be detected without physically separating hybridized and unhybridized forms of the labeled probe. Self-reporting probes, including probes such as molecular beacons that comprise fluorophore and quencher moieties, are highly preferred for both soluble and immobilized probe formats of the invention disclosed herein.

Examples of some homogeneous detectable labels that may be used to practice the invention have been described in detail by Arnold et al., U.S. Patent No. 5,283,174; Woodhead et al., U.S. Patent No. 5,656,207; and Nelson et al., U.S. Patent No. 5,658,737. Preferred labels for use in homogenous assays include, but are not limited to, chemiluminescent compounds (e.g., see Woodhead et al., U.S. Patent No. 5,656,207; Nelson et al., U.S. Patent No. 5,658,737; and Arnold, Jr., et al., U.S. Patent No. 5,639,604). Preferred chemiluminescent labels are acridinium ester ("AE") compounds, such as standard AE or derivatives thereof (e.g., naphthyl-AE, ortho-AE, 1- or 3-methyl-AE, 2,7-dimethyl-AE, 4,5-dimethyl-AE, ortho-dibromo-AE, ortho-dimethyl-AE, meta-dimethyl-AE, ortho-methoxy-AE, ortho-methoxy(cinnamyl)-AE, ortho-methyl-AE, ortho-fluoro-AE, 1- or 3-methyl-ortho-fluoro-AE, 1-or 3-methyl-meta-difluoro-AE, and 2-methyl-AE). Synthesis and methods of attaching labels to nucleic acids and detecting labels are well known in the art (e.g., see Sambrook et al., Molecular Cloning, A Laboratory Manual, 2nd ed. (Cold Spring Harbor Laboratory Press, Cold Spring Harbor, NY, 1989), Chapter 10; Nelson et al., U.S. Patent No. 5,658,737; Woodhead et al., U.S. Patent No. 5,656,207; Hogan et al., U.S. Patent No. 5,547,842; Arnold et al., U.S. Patent No. 5,283,174; Kourilsky et al., U.S. Patent. No. 4,581,333; and Becker et al., European Patent App. No. 0 747 706). Preferably, detection probes labeled with chemiluminescent AE compounds are attached to the probe sequences via a linker substantially as described in U.S. Patent No. 5,585,481; and in U.S. Patent No. 5,639,604, particularly as described at column 10, line 6 to column 11, line 3, and in Example 8.

As illustrated herein, the dynamic range of a hybridization assay that quantifies the amount of an analyte polynucleotide in a sample may be extended by using a plurality of either soluble or surface-immobilized probes. Features of immobilized probes that are useful in these applications, including quantitative probe arrays, are discussed below. Two or more soluble probes that can be used in combination with each other in extended dynamic range assays will exhibit different measurable interactions with the analyte polynucleotide. Examples of these different measurable interactions include the kinetics of the different probe:target binding interactions, and the affinities of the different probes for the analyte polynucleotide. Differences in affinities can conveniently be indicated by different melting temperatures (Tms) for the different probe:target hybrid duplexes.

Soluble probes useful in affinity-shifted probe reagents can be substantially linear or can possess secondary structure. Secondary structure in the soluble probes may comprise sequences which fall outside the sequence of the probe that hybridizes the analyte polynucleotide through complementary base pairing interactions. For example, a soluble probe useful for practicing the invention may include a target-complementary sequence which is complementary to an analyte sequence contained within the analyte polynucleotide, and an additional sequence which is capable of intramolecular base pairing with that target-complementary sequence to form a duplex region having a length of 5 base pairs, or more preferably 5-10 base pairs.

When the amount of analyte polynucleotide is in excess of the amount of affinity-shifted probes, the sequences of the different probes contained in the affinity-shifted probe reagent can either have overlapping or non-overlapping sequences. In the context of the present invention, "overlapping sequences" in two polynucleotide probes are sequences that have the ability to compete with each other for binding through complementary base pairing to the same target polynucleotide sequence. If only duplex hybrids are formed by complementary base pairing of the probes and target, then at least a portion of only one of the two probes that share overlapping sequences will be capable of hybridizing with the target at a single time under conditions of probe saturation. For example, if a first probe is capable of hybridizing nucleotide positions 1-25 of a target polynucleotide, and if a second probe is capable of hybridizing nucleotide positions 20-44 of the same target polynucleotide, then the two probes would be said to have overlapping sequences because nucleotide positions 20-25 of the target polynucleotide are in common between the two probes. When two probes cannot simultaneously hybridize over their entire lengths to the same target polynucleotide sequence because at least one nucleotide position in each probe is complementary to a single position within the analyte polynucleotide sequence, the two probes are said to have the potential to "compete" with each other for binding to the target polynucleotide.

The probes used for conducting an extended dynamic range assay can have identical or different nucleobase sequences, as long as at least one structural feature distinguishes the two probes. For example, the probes can differ in their target-complementary sequences. The target-complementary sequence of one probe can overlap the target-complementary sequence of another probe, or the target-complementary sequence one probe can be a subset of contiguous bases representing the target-complementary sequence of another probe. This latter case would be exemplified when the sequence of one probe is entirely contained within the sequence of another probe that is used in the assay. Alternatively, the probes can have different secondary structures that result from the presence of a nucleobase sequence which falls outside the target-complementary sequence of the probe, but which is able to form a stem-and-loop or hairpin structure that involves a portion of the target-complementary sequence of the probe. According to still another alternative, the probes used in the extended dynamic range assay can have identical nucleobase sequences but differ by the relative presence or absence of nucleic acid analog constituents, including analogs of the backbone, the sugars and/or the bases that make up the probes. Any of these structural differences may be used to create pairs or sets of probes that exhibit different measurable binding interactions with the analyte polynucleotide being quantified in an extended dynamic range assay.

In a preferred embodiment of the extended dynamic range assay, one of the probes contains at least one modified nucleotide which, compared with another probe lacking the modified nucleotide, increases or decreases the Tm of a hybrid duplex between the modified probe and the analyte polynucleotide that is being quantified in the hybridization assay. Such modified probes include oligonucleotides containing at least one 2'-O-methylribofuranosyl moiety joined to a nitrogenous base. However, other modifications which increase or decrease the Tm of a modified probe:target hybrid would reasonably be expected to contribute to differences in the rate of hybridization as well (see Majlessi et al., Nucleic Acids Res. 26:2224 (1998)). Such modifications may similarly occur at the 2' position (or other positions) of the deoxyribofuranosyl or ribofuranosyl moiety (such as 2' halide substitutions), on the nitrogenous bases (such as N-diisobuiylaminomethylidene-5-(1-propynyl)-2'-deoxycytidine; a cytidine analog, or 5-(1-propynyl)-2'-deoxyuridine); a thymidine analog, or in the linkage moiety. Thus, while specific reference is made herein to 2'-methoxy modifications, it is to be understood that other modifications leading to an increased or decreased Tm of a modified probe:target hybrid over a hybrid containing an unmodified probe of identical base sequence would be expected to be similarly useful for making probes targeted to the same analyte polynucleotide with different measurable binding interactions.

Modifications involving the substitution of a methoxy group at the 2' sugar position are particularly preferred for distinguishing probes that are used for extended dynamic range assays that quantify analyte polynucleotides comprising RNA. These 2'-modified oligonucleotides display a preference for RNA over DNA targets having a sequence identical to the RNA target (but having T substituted for U), with respect to both Tm and hybridization kinetics. If a particular probe has a given affinity for an analyte polynucleotide, then substitution of nucleotide analogs having modifications at the 2' position of the deoxyribofuranosyl (or ribofuranosyl) ring can alter the affinity of the resulting probe relative to the starting probe, for a complementary analyte polynucleotide. For this reason, probes distinguished by the presence and absence of 2'-methoxy analogs are very useful for carrying out extended dynamic range hybridization assays that quantify the amplicon products of transcription-based nucleic acid amplification reactions.

Examples of backbone analogs that may be used for structurally distinguishing two probes that may be used in connection with the present invention include "peptide nucleic acids" (PNAs). These are compounds comprising ligands linked to a peptide backbone rather than to a phosphodiester backbone. Representative ligands include any of the four main naturally occurring DNA bases (i.e., thymine, cytosine, adenine or guanine) or other naturally occurring nucleobases (e.g., inosine, uracil, 5-methylcytosine or thiouracil) or artificial bases (e.g., bromothymine, azaadenines or azaguanines, etc.) attached to a peptide backbone through a suitable linker. The PNAs are able to bind complementary ssDNA and RNA strands. Methods for making and using PNAs are disclosed in U.S. Patent No. 5,539,082.

If the two or more soluble probes used in an extended dynamic range hybridization assay are molecular beacons, then those molecular beacons will hybridize to the same analyte polynucleotide with different measurable interactions. As indicated herein, these different measurable interactions result from modifying the structure of one molecular beacon relative to the other. Indeed, the two or more soluble molecular beacons can differ from each other in the manner described below in connection with molecular beacons that are useful in the production of quantitative probe arrays. For example, the soluble molecular beacons can differ from one another in the sequences of their target-complementary loop regions or in their stem regions. The target-complementary loop region of one soluble molecular beacon may be a subset of contiguous bases contained within a larger sequence represented by the target-complementary loop region of a second molecular beacon used in the same assay. Alternatively, the molecular beacon species used in an extended dynamic range assay may hybridize overlapping sequences contained in the analyte, as long as those sequences are contained within the same polynucleotide. Of course, the two or more soluble molecular beacons may differ only, or additionally, in their stem regions to result in a set of probes that interact with an analyte polynucleotide in a way that gives rise to different measurable interactions. One molecular beacon having a stem sequence characterized by a higher Tm will bind less tightly to a target sequence when compared with a different molecular beacon having an identical target-complementary loop sequence and a stem region characterized by a lower Tm. Thus, two or more molecular beacons that differ from each other in their stem sequences and/or target-complementary loop sequences would be useful in combination with each other as soluble probes for extending the dynamic range of a hybridization assay. In certain preferred embodiments of the invention the label present on one of the molecular beacons used in the hybridization assay is not distinguished from the label present on another of the molecular beacons used in the same assay during a detection step subsequent to a probe hybridization step. This may be accomplished by using the same label on each probe, or by using labels that are sufficiently similar to each other that they are detectable using the same apparatus during the detection and measurement procedure.

### General Features of Immobilized Probes that are Useful in Quantitative Probe Arrays

Quantitative probe arrays having an extended dynamic range employ at least two probes, preferably self-reporting probes such as molecular beacons, that are able to hybridize the same species of analyte polynucleotide, and that have different measurable interactions with the analyte polynucleotide. As indicated above, these different measurable interactions result from modifying the structure of one probe relative to the other. For example, different molecular beacon species in an array may differ in the sequences of their target- . complementary loop regions or in their stem regions. The target-complementary loop region of one molecular beacon in the array may be a subset of contiguous bases contained within a larger sequence represented by the target-complementary loop region of a second molecular beacon in the same array. It is also possible for at least two molecular beacon species in the quantitative probe array to hybridize overlapping target regions, as long as those target regions are contained within the same polynucleotide. In the context of the invention, and as indicated above, when two molecular beacons have target-complementary sequences that overlap, it is meant that the two sequences would not be capable of simultaneously hybridizing over their entire lengths to the same target polynucleotide. Indeed, the target-complementary sequences of the two probes can share as few as one nucleotide position in common, but also can be identical over their entire lengths to be considered "overlapping," Molecular beacons that are useful for extending the dynamic range of a hybridization assay conducted using a quantitative probe array are not required to have different target-complementary loop sequences.

Indeed, one molecular beacon having a stem sequence characterized by a higher Tm will bind less tightly to a target sequence when compared with a different molecular beacon having an identical target-complementary loop sequence and a stem region characterized by a lower Tm. As illustrated below, these possibilities can be used to produce quantitative probe arrays that are useful for quantifying analyte polynucleotides or amplicons. Of course, molecular beacons that differ from each other in both their stem sequences and target-complementary loop sequences would also be useful in connection with the present invention.

The affinity and/or hybridization rate for a molecular beacon binding its target polynucleotide generally increases as the length of the loop portion of the molecular beacon is increased, or as the stability of the stem portion of the molecular beacon is decreased. When the concentration of a target polynucleotide to be quantified by hybridization using a quantitative probe array is low, molecular beacons having higher affinities for the target will bind in preference to molecular beacons having lower affinities. Accordingly, molecular beacons differing in the lengths of their target-complementary loop regions will bind the target polynucleotide to differing extents that depend on the concentration of the target in solution. Thus, the relative binding response of a set of two or more molecular beacons having different target-complementary loop sequences able to hybridize a single polynucleotide target, or of two or more molecular beacons having identical loop sequences but differing in their stems, can be used to quantify target polynucleotides over an extended dynamic range.

In general, the extent of the probe-target interaction increases as the affinities of molecular beacon probes for their polynucleotide targets increases. This correlation has been exploited in the present invention so that arrayed sets of molecular beacons having different affinities for the same target polynucleotide exhibit quantitatively different levels of target binding. Thus, a target polynucleotide may be quantified by comparing the relative hybridization signals of at least two molecular beacon probes that have different affinities for the same target polynucleotide.

The fact that individual species of molecular beacons in a quantitative probe array may employ a common fluorophore/quencher combination simplifies detection procedures. This is because optical measurements of hybridization signals can be carried out at uniform excitation and emission wavelengths, thereby simplifying the requirements for analytical instrumentation.

### Nature of Analyte Polynucleotides that are to be Quantified

Analyte polynucleotides that can bind and stimulate detectable responses from soluble or immobilized probes can be quantified using the procedures described herein. Analyte polynucleotides may be quantified directly when a test sample contains sufficiently high numbers of these molecules to generate a detectable signal. Alternatively, an analyte polynucleotide present in a test sample at exceedingly low levels may serve as a template in a nucleic acid amplification reaction that produces analyte amplicons. The resulting amplicons may then be detected using either soluble probes or immobilized probes, such as probes immobilized in a quantitative probe array, and information about the pre-amplification amount of analyte polynucleotide in the test sample determined from the quantitative results.

Regardless of whether soluble probes or immobilized probes are used for quantifying analyte polynucleotides according to the methods disclosed herein, those probes will naturally interact most efficiently with single-stranded targets. Accordingly, it is preferred that analyte polynucleotides or analyte amplicons which are to be quantified are in single-stranded form when they hybridize the affinity-shifted probes of the invention. Thus, double-stranded nucleic acids may require heat denaturation prior to hybridization with probes of the invention.

The quantitative methods described herein can be used for conducting amplification reactions regardless of the origin of the analyte polynucleotide. Preferred analyte polynucleotides include nucleic acids from disease-causing organisms, including viruses, bacteria, fungi and protozoa. Examples of highly preferred analyte polynucleotides from viruses are nucleic acids from the human immunodeficiency viruses (MIV-1 and HIV-2), the hepatitis B virus (HBV), the hepatitis C virus (HCV), and human Parvovirus B19. Preferred analyte polynucleotides from bacteria, fungi and protozoa that can be quantified according to the methods disclosed herein include the ribosomal RNAs (rRNA). Examples of bacteria that are highly preferred as sources of analyte polynucleotides that may be detected using quantitative probe arrays include *Chlamydia trachomatis* (Gram-negative cells that are obligate intracellular organisms), members of the genus Campylobacter (*C*. *jejuni, C*. *coli, C*. *laridis*), members of the genus Enterococcus (*E*. *avium, E*. *casseliflavus, E*. *durans*, *E*. *faecalis, E. faecium, E. gallinarum, E. hirae, E. mundtii, E. pseudoavium, E. malodoratus, and E. raffinosus), Haemophilus influenzae, Listeria momocytogenes, Neisseria gonorrhoeae, Staphylococcus aureus, Group B Streptococci, Streptococcus pneumoniae, Mycobacterium tuberculosis, Mycobacterium avium, Mycobacterium intracellulare, Mycobacterium gordonae*, *Mycobacterium kansasii*. Examples of fungi that are highly preferred as sources of analyte polynucleotides include: *Blastomyces dermatitidis*, members of the genus Candida (*C*. *albicans*, *C*. *glabrata, C*. *parapsilosis, C*. *diversus, C*. *tropicalis, C*. *guilliermondii*, *C*. *dubliniensis*), *Histoplasma capsulatum*, *Coccidioides immitis*. Examples of protozoa that are highly preferred as sources of analyte polynucleotides include blood and tissue protozoa, such as members of the genus Plasmodium (*P*. *malariae*, *P*. *falciparum*, *P*. *vivax*), as well as protozoa that infect the gastrointestinal tract such as *Giardia lamblia* and *Cryptosporidium parvum*.

Affinity-shifted probe reagents, including quantitative probe arrays, also can be used for quantifying nucleic acids that are of human origin, such as mRNAs that are over-expressed or under-expressed in disease states, including cancers. One example of a gene that is present at an increased copy number in breast and ovarian adenocarcinomas is the HER-2/neu oncogene which encodes a tyrosine kinase having certain features in common with the epidermal growth factor receptor (EGFR). U.S. Patent No. 4,968,603 describes the value of measuring the increased copy number of the HER-2/neu gene, or the HER-2/neu mRNA as a tool for determining neoplastic disease status. Thus, for example, the method described herein can be employed in quantitative nucleic acid amplification protocols whereby the cellular content of HER-2/neu polynucleotides is determined.

Indeed, the methods described herein are broadly applicable and may easily be adapted to procedures for quantifying any given analyte polynucleotide in a test sample.

### Use of Standard Control Curves

Measurements obtained using affinity-shifted probe reagents, including quantitative probe arrays, conventionally are compared with one or more "standard curves" so that the amount of analyte polynucleotide target present in a test sample can be determined. Standard curves are produced by contacting the soluble or immobilized probes with known amounts of the analyte polynucleotide. When the probes are immobilized probes, it is preferred that the immobilized probes are self-reporting probes, such as molecular beacons. Regardless of whether soluble or immobilized probes are employed, these procedures can be carried out directly on test samples (no amplification) or with amplicons produced in nucleic acid amplification reactions.

If an analyte polynucleotide in a test sample is to be quantified directly, then a series of hybridization reactions that include known amounts of an analyte polynucleotide standard can be run in parallel with a hybridization reaction that includes the test sample which is suspected of containing analyte polynucleotides. For example, if the procedure involves either soluble affinity-shifted probe reagents or quantitative probe arrays prepared in the wells of a microtiter plate, then separate aliquots of the probe reagent, or separate wells of the microtiter plate can be used for hybridizing each different amount of polynucleotide standard, with one aliquot or microtiter well being used for hybridization of the test sample. At the conclusion of a predetermined time, detectable signals associated with hybridized probe may be measured for each amount of polynucleotide standard used in the procedure, and the result plotted on a graph. In certain preferred embodiments, wherein a single species of probe is immobilized at a plurality of spots in an array, detectable signals from different spots representing the same probe may be averaged and then plotted on a graph. Optionally, the numerical results obtained using different probes at any particular amount of standard may be mathematically compared with each other (for example to form a ratio), and that result plotted graphically. For example, if a quantitative probe array comprises five samples of a first probe having a first affinity for a target polynucleotide, and five samples of a second probe having a second affinity for a target polynucleotide, then fluorescent signals from each set of five samples may be averaged separately and the resulting two values compared thereafter.

It should be clear from the foregoing discussion that standard curves represent tools for determining the amount of analyte polynucleotide present in the test sample. More particularly, the hybridization signal obtained in the trial conducted using the test sample can be located on a first axis of the standard curve, and the corresponding amount of analyte polynucleotide present in the test sample determined from a second axis of the same standard curve. Sample standard curves are described in the working Examples herein.

Since nucleic acid amplification reactions can feature quantitative relationships between the number of analyte polynucleotides input into the reaction and the number of analyte amplicons synthesized, the pre-amplification amount of an analyte polynucleotide present in a test sample can also be determined using a standard curve produced using affinity-shifted probe reagents, including quantitative probe arrays. For example, a plurality of amplification reactions containing known amounts of analyte polynucleotide standard can be carried out in parallel with an amplification reaction prepared using a test sample containing an unknown number of analyte polynucleotides. Preferred amplification methods include Transcription Mediated Amplification (TMA) reactions, Nucleic Acid Sequence Based Amplification (NASBA) reactions, Strand Displacement Amplification (SDA) Reactions, Ligase Chain Reactions (LCR), and Polymerase Chain Reactions (PCR). Transcription Mediated Amplification is highly preferred.

Of course, standard control curves can be prepared using either soluble or immobilized versions of affinity-shifted probes. If the procedure involves hybridization of soluble affinity-shifted probes, then nucleic acid amplification reactions may first be conducted using an aliquot of the test sample or known amounts of standard analyte polynucleotide together with reagents adequate to promote the amplification reaction. At the conclusion of the amplification reaction, an affinity-shifted probe reagent that includes at least two soluble probes that hybridize the analyte amplicon with different affinities can be hybridized to the amplification products. If the procedure involves nucleic acid amplification in contact with probes immobilized in a microtiter plate format, then different wells, each comprising arrayed sets of at least two molecular beacon probes able to bind the analyte amplicon, may receive an aliquot of the test sample or known amounts of standard analyte polynucleotide together with reagents adequate to promote the amplification reaction. At the conclusion of the amplification reaction, measured fluorescent signals from spots in each well can then be determined. A graphical standard curve having pre-amplification amounts of the analyte polynucleotide standard plotted on a first axis and corresponding post-amplification fluorescent signals on a second axis is then prepared. The post-amplification signal measured for the test reaction is then located on the post-amplification axis of the standard curve. The corresponding value on the other axis of the curve represents the pre-amplification amount of analyte polynucleotide that was present in the test reaction. Ratios of the averaged, measured signals optionally may be determined and plotted on a graph for comparison. Thus, determining the number of molecules of analyte polynucleotide present in a test sample is accomplished by consulting the standard curve, or more particularly by comparing the quantitative results obtained for the test sample with the standard curve.

Notably, when the precision of results obtained using affinity-shifted probe reagents is acceptably high, then it is desirable to have prepared in advance a standard curve so that it is unnecessary to prepare a curve each time an analytical procedure is performed. The results used for producing standard curves may be stored in printed graphic or electronic form so that comparison of test results with the results obtained using the standards may be automated. This approach advantageously eliminates the need to carry out laboratory procedures using known amounts of a polynucleotide standard each time a test sample is to be analyzed. In a preferred embodiment of the invention, the standard results, whether in tabular or graphic form, are stored electronically in a memory device of an analytical instrument. Results obtained from analysis of a test sample may be compared with the standard curve stored in the memory device to determine the amount of analyte polynucleotide present in the test sample.

### Instrumentation for Making and Using Quantitative Probe Arrays

Microarray fabrication technology is now sufficiently advanced that devices useful for creating arrays by deposition of pre-formed nucleic acids are commercially available. For example, mechanical or robotic microspotting devices may be purchased from GeneMachines (San Carlos, CA), BioRobotics Ltd. (Cambridge, UK), Cartesian Technologies, Inc. (Irvine, CA), and Packard BioScience Co. (Meriden, CT). These devices enable one to produce arrayed spots of molecular beacons or other polynucleotide on planar surfaces.

Alternative array formats which may be used in connection with the present invention involve optical fibers or microspheres. For example, optical fiber-based nucleic acid detectors of the type described in published International Application PCT/US00/13753, entitled, "Combinatorial Decoding of Random Nucleic Acid Arrays" represent non-planar array formats that easily can be adapted to accommodate quantitative probe arrays. Compositions and methods employing oligonucleotide probes coupled to microspheres which can be adapted for use in connection with the present invention are disclosed in U.S. Patent No. 6,057,107.

Devices for detecting fluorescent signals emitted by arrayed molecular beacons also are commercially available. For example, scanners for analyzing the results of hybridized arrays may be purchased from Molecular Dynamics (Piscataway, NJ).

### Devices Incorporating Quantitative Probe Arrays

Devices that detect and quantify analyte polynucleotides or analyte amplicons in accordance with the methods disclosed herein comprise a plurality of hybridization probes that are characterized by certain structural and functional relationships to each other. In a convenient format, the probes are disposed as arrays immobilized onto solid supports. In its simplest form, one embodiment of a quantitative probe array device has immobilized two different probes that hybridize the same analyte polynucleotide with different measurable interactions. Significantly, the amount of any probe contained in the quantitative probe array advantageously may be lower than the upper limit amount of analyte polynucleotide that is to be detected in a hybridization procedure. For instance, Example 4 herein describes the use of 20 pmols of each of 5 different probes for measuring amounts of a synthetic analyte polynucleotide as high as 2,000 pmols. This represented an amount of analyte polynucleotide that was 100 fold greater than the amount of probe used in the assay. When the device is to be used for quantifying an analyte polynucleotide following an amplification procedure, it is preferred that a positive control probe is included in the array. A negative control probe optionally may also be included in the array. The different probes in the array are immobilized onto a solid support, and the immobilized probes preferably are in fluid communication with each other. The number of different quantitative hybridization probes that are complementary to a single analyte polynucleotide, the structural features which may confer different measurable interactions between the quantitative probes and the analyte polynucleotide, and the number of different analyte polynucleotides which may be detected and quantified are all aspects of the invention.

Quantitative probe arrays include at least two different hybridization probes for detecting and quantifying an analyte polynucleotide. Indeed, it is contemplated that the quantitative probe array may include two different probes, three different probes, four different probes, or five different probes, all of these different probes being complementary to the same analyte polynucleotide that is to be quantified. Of course, devices that are intended to be used for multiplex detection of different analyte polynucleotides will have proportionately greater numbers of different hybridization probes. For example, a quantitative probe array for quantifying two different analyte polynucleotides will include four different hybridization probes, wherein two of the probes are complementary to the first analyte polynucleotide and the remaining two probes are complementary to the second analyte polynucleotide. Of course, any number of different probes may be included in the device. However, if an assay for quantifying an analyte polynucleotide is to have an extended dynamic range beyond the quantitation range provided by a single probe, then more than one hybridization probe will be used for hybridizing the analyte polynucleotide that is to be detected and quantified.

When a quantitative probe array includes more than one different hybridization probe that is able to bind the analyte polynucleotide, each of the different probes will have a different measurable binding interaction with the analyte polynucleotide. The different measurable interactions which distinguish the probes may result from differences in the affinities with which the different probes and the analyte polynucleotide interact. Since the Tm of a probe:target complex, or the temperature at which 50% of probe:target complexes become denatured, is a reflection of the affinity of the probe for the target, this easily determined parameter can be used for establishing that candidate probes have different measurable binding interactions with the analyte polynucleotide. Methods of determining the Tm of a probe:target complex are well known in the art. Thus, Tm is an example of a parameter that can be used for assessing the different measurable binding interactions that distinguish probes that can be used for quantifying analyte polynucleotides in extended dynamic range assays as described herein. This same test can be applied for affinity-shifted probes that can be used in soluble probe formats as well as in immobilized probe formats.

Structural features that lead to different measurable interactions between different probes and a single analyte polynucleotide may involve the length of the probe sequence which is complementary to the analyte polynucleotide. For example, a first probe may differ from a second probe by hybridizing only a subset of the target sequence that is hybridized by the second probe. One probe may hybridize an internal subset of the sequence hybridized by the other probe. Alternatively, the probes may hybridize overlapping sequences. Different measurable binding interactions also could be achieved by employing identical target-complementary probe sequences, where one of the probes additionally contains a sequence complementary to a subset of the target-complementary probe sequence. In this way the two probes will be distinguished by different secondary structures.

Still other quantitative probe arrays will employ probes with identical nucleobase sequences, but that differentially include nucleotide analog substitutions. For example, a probe comprising 2'-OMe analogs will have increased affinity for its target analyte polynucleotide when compared with a probe prepared using only deoxyribonucleotides. Probes that will be useful for conferring an extended dynamic range in a quantitative probe array will give non-identical sigmoid curves on a plot of hybridized probe versus input target polynucleotide in a procedure wherein different amounts of analyte polynucleotide standard are hybridized with the individual probes. This procedure, which is illustrated by the working Examples herein, can be used as a convenient but stringent test for selecting probes that have different measurable interactions with the same analyte polynucleotide.

Quantitative probe arrays that are used in conjunction with nucleic acid amplification reactions typically will include "control" probes for verifying the integrity of an amplification reaction. Conventionally, the signal generated by a positive control probe must be above some threshold level for the amplification reaction to be considered valid. For example, a reaction for amplifying an analyte polynucleotide additionally may include an internal control template that can be amplified by the same primer set that is used for amplifying the analyte polynucleotide. The internal control template may be a "pseudo target" that is amplified by the same primer set that amplifies the analyte polynucleotide, but that contains a scrambled internal sequence that allows for independent detection using a hybridization probe different from the probe that is used for detecting the analyte amplicon (see published International Patent Application No. WO 01/07661). Thus, the positive control probe in the quantitative probe array will hybridize the amplification product of the internal control template, but will not hybridize the amplicon corresponding to the analyte polynucleotide. As indicated above, some quantitative probe arrays may include a negative control probe that hybridizes an irrelevant target, and not the analyte polynucleotide or analyte amplicon.

The individual probe species of the invented quantitative probe array preferably are immobilized to a solid support. While different solid supports may be used, certain preferred embodiments of the invention employ the flat, inner surfaces of microtiter wells as the solid support for receiving the immobilized probes. The receiving surface may comprise either glass or plastic. The different probes used for quantifying the analyte polynucleotide over an extended dynamic range may harbor detectable labels that are distinguishable or indistinguishable from each other. In preferred embodiments, the different probes harbor the same detectable label, but may harbor different detectable labels that are not distinguished from each other by the apparatus that is used for detecting and measuring hybridization signal strength. In certain preferred embodiments of the invention, probes harboring the same detectable label are immobilized independently at different spots within an array. In these instances, the probes may be distinguished from each other. In other preferred embodiments, different probes harboring the same detectable label, or different detectable labels that are not distinguished from each other are immobilized at a single spot in the array. Which of these approaches is followed is a matter of choice for carrying out the invention because each of these approaches will give good quantitative results over an extended dynamic range. In all of these cases, when the probes of the invention are molecular beacons, it is possible to use the same fluorophore and quencher pair for all of the different molecular beacons in the quantitative probe array.

As indicated above, certain preferred devices incorporating quantitative probe arrays are prepared using glass or plastic microtiter plates, slides or similar devices as solid supports. The flat, inner surfaces of standard 96-well or 384-well microtiter plates are appropriately suited for preparing arrays of spatially separated samples using commercially available arraying devices. We have disposed up to 100 spatially separated samples in an array configuration on the flat, inner surface of a single well of a 96-well microtiter plate. The rigidly fixed arrangement of wells in the microtiter plate advantageously permits convenient manual or automated addition of liquid reagents, and additionally permits nucleic acid amplification reactions and detection of analyte polynucleotides or analyte amplicons to be carried out in a single reaction vessel. Moreover, the planar bottom surfaces of microtiter plates are compatible with commercially available detection instruments that can be used for quantifying fluorescent signals emitted from individual spots in the array. One advantage of creating quantitative probe arrays in the wells of microtiter plates relates to the ability to carry out nucleic acid amplification reactions in the arrayed wells. This eliminates any need for transferring an amplified sample from a first reaction container to a second container for the quantitative portion of the assay. Of course, when quantitative probe arrays are created using microtiter wells, the spotted probes of an array will be in fluid communication with each other. This advantageously allows each probe in the array to be subjected to the same amplification reaction.

In preferred embodiments of the invention, immobilized probes of the quantitative probe arrays are self-reporting probes, and in highly preferred embodiments the self-reporting probes are molecular beacons. The probes of the quantitative probe array may be immobilized to the solid support either through covalent or non-covalent coupling. When the probes are molecular beacons, the molecular beacons may be coupled via one of the two arm sequences, through an arm-loop junction where one of the arms meets the target-complementary loop sequence, or through the target-complementary loop sequence. While acceptable results may be achieved using any one of these approaches, it is highly preferred to immobilize molecular beacons to the solid support from a position located within the target-complementary loop sequence. More particularly, it is highly preferred to couple molecular beacons of the invention to solid surfaces through a non-nucleotide linker which is disposed in the sequence of the target-complementary loop. This approach gave particularly good results by allowing the probe to remain in the closed conformation in the absence of a complementary target polynucleotide, thereby producing low background signals. Moreover, this configuration facilitated efficient interaction with the complementary target polynucleotide and promoted strong signal generation. Thus, immobilization of a molecular beacon through the target-complementary loop advantageously gave low backgrounds and efficient signal generation in response to target binding.

Preferred methods for non-covalently attaching molecular beacons to solid surfaces include the combination of biotinylated molecular beacons and avidin-, or streptavidin-coated surfaces. Biotin can be joined to the molecular beacon through a non-nucleotide linker, such as that disclosed by Arnold et al., in U.S. Patent No. 5,696,251. Preferred methods for covalently attaching molecular beacons to solid supports also involve linkage through the target-complementary loop region of the molecular beacon, for example using this or a similar non-nucleotide linker.

Covalent attachment of probes to solid surfaces may be accomplished using any of a number of different techniques that will be familiar to those having an ordinary level of skill in the art. For example, a description of coupling chemistry that may be used for joining the molecular beacon to the surface can be found in U.S. Patent No. 6,171,797.

In accordance with certain embodiments of the invention, and to facilitate statistical analysis of quantitative results, it is preferred for devices incorporating quantitative probe arrays to employ redundant collections of immobilized probes. In some embodiments
wherein the probes are molecular beacons, each molecular beacon species is immobilized in the array independent of other species of molecular beacon. Indeed, a single species of molecular beacon may be disposed at multiple positions within the array. Detection of hybridization signals from a plurality of independent spots in the array facilitates statistical analysis of the results, thereby improving their reliability. Preferably, the same molecular beacon is present 2-10 times (meaning at 2-10 loci or spots), still more preferably 2-5 times, and still more preferably 3-5 times. It is highly preferred for the same molecular beacon to be present 5 times, still more preferably 4 times, still more preferably 3 times, and most preferably 2 times in a single array. However, in other embodiments each species of molecular beacon may be present only once in a single array.

Still other structural features of quantitative probe arrays relate to optional controls for monitoring spot-to-spot variation between replicate spotted probes within a single probe array. More particularly, when replicate spotting is used to provide a basis for statistical analysis of hybridization results, it is desirable to have some means for correcting the data to normalize the effects of differential spotting efficiency. Stated differently, it is desirable to be able to measure the amount of a probe that is present at a spot in an array so that any signal read from that spot can be normalized for the amount of probe that is present. This may be accomplished either by co-immobilizing the probe and a second oligonucleotide that may be independently detected, or by incorporating into the structure of the probe a second label that can be detected independent of the target-dependent signal that results from hybridization of the probe and its complementary analyte polynucleotide. For example, if the immobilized probe is a molecular beacon which comprises a fluorophore-quencher pair, then the molecular beacon may be constructed to incorporate a different fluorophore which is not quenched by the quencher and which has an emission spectrum that is distinguishable from the fluorophore which indicates probe binding. These examples illustrate how quantitative probe arrays can be constructed so that numerical indications about the amount of immobilized probe present at a spot in an array may be easily determined.

Figure 4 schematically illustrates arrayed microtiter wells representing various devices that incorporate quantitative probe arrays. Figure 4A shows an array of two spots, each representing a different probe able to hybridize a single analyte polynucleotide. Figure 4B shows the combination of the two probes from panel A combined at a single locus. Figure 4C shows an array that includes independently immobilized first and second probes that hybridize a single analyte polynucleotide, and additionally includes a positive control probe that hybridizes an internal control amplicon. This embodiment of the quantitative probe array would be suited for use in connection with nucleic acid amplification assays that include an internal control template for verifying integrity of the amplification reaction. Figure 4D shows an embodiment similar to that illustrated in panel C, but which additionally includes a negative control probe. This embodiment would be suited for use in connection with nucleic acid amplification assays that include an internal control template for verifying integrity of the amplification reaction, and that are sufficiently precise that it is unnecessary to carry out parallel amplification reactions using analyte polynucleotide standards. Figure 4E shows an embodiment similar to that illustrated in panel C, but which additionally includes a set of two probes for quantifying a second analyte amplicon. This embodiment would be suited for multiplex amplification systems wherein two different analyte polynucleotides may be amplified, detected and quantified in a single nucleic acid amplification reaction.

### Co-Immobilizing Different Probes at a Common Locus in an Array

Certain quantitative probe arrays that are useful for conducting assays characterized by extended dynamic ranges have immobilized at a single locus in the array more than one probe species that hybridizes the analyte polynucleotide. For example, it may be desirable to immobilize two probes, each having a hybridization profile different from the other, at a single spot in a quantitative probe array. Of course, these different hybridization profiles will result from different measurable binding interactions with the same analyte polynucleotide. Different Tms characterizing the probe: analyte interactions are examples of different measurable binding interactions. The detectable label that indicates binding of the analyte polynucleotide and the immobilized probe may be the same or different for the co-immobilized probes. If the co-immobilized probes are molecular beacons, then the different molecular beacons may have the same fluorophore-quencher pairs. Preferably, the co-immobilized probes share detectable labels that are indistinguishable, or that are not distinguished from each other in a step for detecting and measuring hybridization signals.

The relative amounts of the different probe species co-immobilized at a single locus in a quantitative probe array may be different from each other. For example, if two molecular beacon species having different measurable binding interactions with the same analyte polynucleotide are co-immobilized at a single locus in an array, then it is possible for the two molecular beacons to be present in proportions that are not equivalent. For example, the first molecular beacon may be present in an amount that is twice as great as the amount of the second molecular beacon at the same spot in the array. In this instance, the first molecular beacon would represent two-thirds of the total amount of immobilized probe at the locus. When two probes are co-immobilized at a single spot in a quantitative probe array, the proportion of the total immobilized probe amount represented by one of the two probes may be about one-half, about one-half to about one-third, or about one-third to about one-quarter of the total immobilized probe on a molar basis.

Quantitative probe arrays having different proportions of more than one probe immobilized at a single locus offer certain advantages when used in connection with nucleic acid amplification reactions. This is particularly true when the amount of probe exhibiting preferential binding at lower amounts or concentrations of target polynucleotide (i.e., the "high affinity probe") is higher than the amount of probe that exhibits binding at higher amounts or concentrations of target polynucleotide (i.e., the "low affinity probe"). When a greater proportion of the high affinity probe is immobilized, the contribution of the hybridization signal from that probe to a composite signal can be emphasized. This is reflected by an increased slope of the sigmoid composite curve representing the total hybridization signal in the region of the curve corresponding to low target amounts. This is important because the greatest uncertainty in amplicon production occurs at low levels of input template. Accordingly, it is an advantage to produce sigmoid plots having slopes that are enhanced in the part of the curve corresponding to low levels of input template. Varying the relative amounts of the different co-immobilized probes provides a way to increase the reliability of quantitative results obtained in nucleic acid amplification reactions, particularly when the starting amount of analyte template is very low.

Of course, quantitative probe arrays having different probe species independently immobilized also may have different relative amounts of probes immobilized at different spots in the array. Again, the proportion of the total immobilized probe amount represented by one of the two probes may be about one-half, about one-half to about one-third, or about one-third to about one-quarter of the total immobilized probe on a molar basis.

### Quantitative Probe Arrays Having Unlabeled Probes

Quantitative probe arrays may be constructed using immobilized polynucleotide probes that are unlabeled. These quantitative probe arrays, which may be used for detecting and measuring the amount of a labeled amplicon synthesized in an *in vitro* nucleic acid amplification reaction, will include two or more immobilized polynucleotide probes that hybridize a single analyte polynucleotide with different measurable binding interactions. The immobilized probes consequently will exhibit different hybridization profiles when hybridized with the analyte polynucleotide. When contacted with a labeled polynucleotide, such as an amplicon synthesized using labeled nucleotide triphosphate or deoxyribonucleotide triphosptates, or labeled primers, the immobilized probes of the quantitative probe array will bind the labeled analyte. Hybridization signals measured for different probes in the array may be compared with a standard curve. In this way it is possible to quantify the starting amount of a polynucleotide that served as a template in an amplification reaction that synthesized detectably labeled amplicons. Of course, a positive control template, such as a pseudo target, also may be included in the amplification reaction and may be detected using an immobilized probe that hybridizes the pseudo target amplicon but not the amplicon that was synthesized using the analyte polynucleotide as a template. In these embodiments the detectable label which is used for quantifying the magnitude of hybridization is provided by the polynucleotide that is to be quantified, rather than by an immobilized probe in the quantitative probe array.

### Methods of Making Affinity-Shifted Probe Reagents / Quantitative Probe Arrays

Affinity-shifted probe reagents and quantitative probe arrays are created by first selecting two or more probes that hybridize the analyte polynucleotide that is to be quantified, subject to the provision that the selected probes hybridize the analyte polynucleotide with different measurable binding interactions. For example, the two probes may exhibit different affinities or Tms when hybridized with the analyte polynucleotide. Conventionally, one of the probes will hybridize the analyte polynucleotide with a higher affinity, while the other probe will hybridize the analyte polynucleotide with a lower affinity. As a consequence, the two probes will have different hybridization profiles for binding the analyte polynucleotide. General features of probes that may be used in soluble reagent and quantitative probe arrays have been described above.

Next, the two or more selected probes are either combined, if the probes are to be used in a soluble reagent format, or immobilized to a solid support. Preferably, the solid support is made of a material which is compatible with conducting a nucleic acid amplification reaction. For example, the solid support may be an individual well contained in a microtiter plate. The immobilized probes may be immobilized uniformly over the inner bottom surface of one of more wells in the microtiter plate, or alternatively may be spotted in an array format. Optionally, the probes are combined prior to the immobilization procedure so that a single spot in the array will contain more than one probe. If the immobilized probes are labeled probes, it is possible, indeed preferred that the different probes contain labels that are not distinguished from each other during a detection step, or are otherwise indistinguishable.

### Kits for Quantifying Polynucleotides

Kits for quantifying an analyte polynucleotide using soluble affinity-shifted probe reagents will include at least two probes that are able to hybridize the analyte polynucleotide with different measurable binding interactions. In a preferred embodiment, these different measurable binding interactions are indicated by different affinities, measurable by different Tms for independent hybrid duplexes formed between the analyte and any of the probes. In another preferred embodiment, the different measurable binding interactions are indicated by different binding kinetics, meaning that one of the probes binds to the analyte polynucleotide faster than another of the probes. The probes may have nucleobase sequences that are non-overlapping, overlapping, or even identical, as long as at least one structural feature distinguishes the probes from each other. For example, the probes may be distinguished by the presence or absence of nucleotide analogs, or by the presence of sequences which promote formation of different secondary structures in the different probes. Further, it is preferred that each of the soluble affinity-shifted probes is labeled with a detectable label. In certain preferred embodiments, the detectable labels are identical to each other. However, in other preferred embodiments, the detectable labels are different from each other but may be detected in a manner that does not distinguish the signals originating from the different labels. In still other preferred embodiments, the labels on the different soluble probes are distinguishable from each other. The kit may further include oligonucleotide primers for amplifying the analyte polynucleotide that is to be quantified. Optionally, the kit may also include a positive control polynucleotide which may serve as a template in an amplification reaction, and which may be a pseudo target. Positive control amplicons preferably would be detectable by hybridization probes different from the probes used for quantifying the analyte polynucleotide. Alternatively, rather than including a positive control polynucleotide, the kit may instead include a polynucleotide standard that can be included in an amplification reaction. Amplicons generated from the polynucleotide standard would be detectable by the same probes used for quantifying the analyte polynucleotide. Under still another alternative, the kit may include both a positive control polynucleotide and a polynucleotide standard. Again optionally, the kit may further include reagents for carrying out a polynucleotide amplification reaction. For example, the reagents may comprise deoxyribonucleotide triphosphates. A DNA polymerizing enzyme, which may be a reverse transcriptase, also can be included. Nucleotide triphosphates and an RNA polymerase are still other reagents that can be included in the kit.

Kits for quantifying polynucleotides using a quantitative probe array will include: a quantitative probe array, and oligonucleotide primers for amplifying analyte polynucleotide. The kit optionally may include either a positive control polynucleotide, which may serve as a template in an amplification reaction, and which may be a pseudo target, or a standard that can be added included in an amplification reaction. In certain kits, both the positive control polynucleotide and the polynucleotide standard will be included. Kits for quantifying polynucleotides following a nucleic acid amplification reaction additionally may include reagents for carrying out the polynucleotide amplification reaction. Reagents included with the kit may comprise deoxyribonucleotide triphosphates. A DNA polymerizing enzyme, which may be a reverse transcriptase, also can be included. Nucleotide triphosphates and an RNA polymerase are still other reagents that can be included in the kit.

### Preferred Embodiments of the Invention

The following Examples illustrate some of the ways for carrying out the invention. Example 1 describes a general procedure for extending the dynamic range of quantitative assays that employ probes for detecting analytes. In this instance, soluble hybridization probes harboring chemiluminescent labels were used for quantifying a model analyte polynucleotide. Example 2 shows how the procedure was used in conjunction with nucleic acid amplification to quantify pre-amplification amounts of analyte polynucleotide that spanned a broad range. In Examples 3 and 4, immobilized probes were spatially separated in different wells of a microtiter plate and so were not in fluid communication with each other. Subsequent Examples describe procedures employing different probes immobilized within the same well of a microtiter plate in an array format. In these procedures the spatially separated and immobilized probes were in fluid communication with each other. Although molecular beacon probes were used in Examples disclosing procedures in array formats, it is to be understood that other types of probes may be used with equally good results, and are embraced within the scope of the invention. For example, the above-described molecular torches represent alternative self-reporting probes that can be used to practice the invention in its various embodiments.

Those having an ordinary level of skill in the art will appreciate that an extended dynamic range is evidenced by an extended linear portion of a sigmoid plot that relates the extent of probe binding and input target amounts. Using nucleic acid hybridization as an example, graphic plots of hybridization signal strength versus input amounts of target polynucleotide will give different curves for different probes that hybridize the same target polynucleotide. The assay that yields a more extensive linear portion of the sigmoid curve will be useful for quantifying a broader range of analyte polynucleotide amounts or concentrations, and so will be characterized by an extended dynamic range.

With reference to quantitative methods employing a plurality of probes capable of binding the same analyte species, maximum quantitative capacity of the assay is achieved when the individual probes have useful quantitative ranges that overlap only minimally. For example, rather than employing two probes capable of detecting an analyte polynucleotide over ranges of 10¹-10³ and 10²-10⁴ units, respectively, it is more desirable to employ two probes having useful detection ranges of 10¹-10³ and 10³-10⁵ units instead. The basis for this feature of the invention will be clear upon reviewing the results under Example 1.

The following Example illustrates how the invention may be used for quantifying analytes over an extended dynamic range using a plurality of different probes that bind the same analyte species with different measurable interactions. The invention may be applied to the quantitation of biological macromolecules, including: proteins, nucleic acids and carbohydrates. In addition, the invention may be applied to the quantitation of biological small molecules, including peptide and steroid hormones. Indeed, the quantitative method disclosed herein may be applied to the quantitation of any analyte for which at least two probes having different measurable interactions. In the following Example, two probes differing in length by 6 nucleotides exhibited different hybridization profiles when hybridized with the same target polynucleotide across a range of target amounts.

Example 1 describes methods used to demonstrate how two different probes, each having binding specificity for the same model analyte polynucleotide and being labeled with indistinguishable chemiluminescent labels, can be used to quantify the analyte polynucleotide over an extended dynamic range. In these procedures the resulting plots represent standard control curves produced using known amounts of input target representing the analyte polynucleotide. The sequence of the model analyte polynucleotide used in this Example was derived from an *E*. *coli* rRNA sequence.

### Example 1

### Extending the Dynamic Range of a Quantitative Assay Using a Plurality of Labeled Probes Having Detectable Labels that are Indistinguishable

To demonstrate the basis of the quantitative approach underlying the present invention, an experiment was conducted using three types of hybridization reaction, each reaction being characterized by its own dynamic range. Target RNA, representing a model polynucleotide in the procedure, had the sequence AUGUUGGGUUAAGUCCCGCAACGAGC (SEQ ID NO:1). A first probe was a synthetic 26-mer DNA molecule having the sequence GCTCGTTGCGGGACTTAACCCAACAT (SEQ ID NO:2), and was labeled with acridinium ester (AE) to a specific activity of 1.31 x 10⁸ rlu/pmole. The second probe was a synthetic 20-mer DNA molecule having the sequence TGCGGGACTTAACCCAACAT (SEQ ID NO:3), and was labeled with AE to a specific activity of 9.37 x 10⁷ rlu/pmole. The first and second probes were labeled with AE moieties between positions 16 and 17, and between positions 10 and 11, respectively. In each case, the AE label was linked to the probe through a non-nucleotide linker, essentially as described by Arnold et al., in U.S. Patent No. 5,696,251. Hybridization reactions having volumes of 80 µl each were carried out for 8 minutes at 65°C in a lithium succinate buffered solution containing lithium lauryl sulfate. Three sets of reactions included amounts of the model analyte polynucleotide ranging from 0 - 200,000 fmol. The first set of hybridization reactions included 0.5 fmol of the 26-mer probe. The second set of hybridization reactions included 0.5 fmol of the 20-mer probe. The final set of hybridization reactions included 0.5 fmol each of the 26-mer probe and the 20-mer probe. At the conclusion of the hybridization reaction, the extent of bound probe was assessed using the adduct protection assay described by Mazumder et al., in Nucleic Acids Res. 26:1996 (1998). In this procedure the chemiluminescence of the hybridization reaction was measured by injection of 100 µl of 14 mM NaSO₃ in 42 mM borate buffer (pH 8.8), followed by injection of 100 µl of a solution of 1.5 M NaOH and 0.12% H₂O₂. Light emission, which reflected the extent of probe bound to the analyte polynucleotide target, was measured in relative light units (RLU) by luminometry.

The graphic results presented in Figure 1 demonstrate that each of the hybridization reactions was characterized by a different useful range for quantifying analyte polynucleotide. More particularly, the 26-mer and 20-mer hybridization probes were independently useful for quantifying analyte polynucleotide over the 10¹-10³ and 10³-10⁵ fmol ranges, respectively. Thus, assays employing the individual probes were capable of measuring analyte polynucleotide amounts over ranges that spanned approximately 100 fold. However, the reaction conducted using the combination of the two probes advantageously exhibited a useful quantitative range of from 10¹-10⁵ fmol of analyte polynucleotide, or a range that spanned approximately 10,000 fold. With respect to the other reactions, the hybridization reaction conducted using a plurality of hybridization probes that bound the same analyte polynucleotide species using probe sequences that overlapped each other, and that differed in length by only 6 nucleotides, advantageously exhibited an extended dynamic range.

As indicated above, the results presented in Figure 1 represent standard control curves prepared using known quantities of an analyte polynucleotide. A parallel hybridization reaction conducted using a test sample containing an unknown amount of analyte polynucleotide also could be carried out, and the results from that procedure compared with the standard curve to determine the amount of analyte polynucleotide contained in the test sample. Although equimolar amounts of two probes were used in the procedure described above, differing amounts of two or more probes also may be used for extending the dynamic range of quantitative assays.

The foregoing results illustrated how two different probes, each harboring indistinguishable labels and binding the same analyte with a different measurable interaction, can be used to quantify the analyte over an extended dynamic range. Although polynucleotide analytes were employed in the exemplary procedure, the principle underlying the quantitative procedure may also be used for quantifying non-polynucleotide analytes.

Although extended dynamic range hybridization assays for quantifying amplicons using soluble probes may be conducted in a real-time format, the following Example describes hybridization assays that were conducted at the conclusion of an amplification reaction. More particularly, in this procedure an extended dynamic range probe reagent that included two soluble, HCV-specific linear oligonucleotide probes was used for quantifying the products of nucleic acid amplification reactions. The two probes were each 22 nucleotides long and had identical nucleobase sequences except for substitution of the DNA/RNA equivalent bases thymine and uracil. A key difference between the two probes was that one comprised deoxyribose sugar moieties that are standard in DNA, and the other comprised analogs having a methoxy group at the 2' position of the ribose (2'-OMe) moieties. The presence of the 2'-OMe analogs increased the Tm of the probe:target hybrid, thereby distinguishing the binding characteristics of the two probes. The affinity of methoxy Probe B for the analyte amplicon was higher than the affinity of deoxy Probe A for the same analyte amplicon. Notably, the deoxy and methoxy probes contained in the extended dynamic range probe reagent are respectively referenced below simply as "Probe A" and "Probe B." The two probe species were either used separately or combined and hybridized with analyte amplicons. Both of the probes were labeled with chemiluminescent labels that were simultaneously detected by luminometry without distinguishing the labels.

Example 2 illustrates how two probes that hybridized identical target sequences within the same analyte polynucleotide were used for quantifying nucleic acid amplification products in an extended dynamic range hybridization assay. A portion of the hepatitis C virus (HCV) genome served as the analyte polynucleotide undergoing amplification in this procedure. The hybridization probes were labeled with chemiluminescent labels that were not distinguished from each other in the luminometry step that detected and quantified the magnitude of probe hybridization.

### Example 2

### Quantitation of HCV Using Nucleic Acid Amplification and an Extended Dynamic Range Hybridization Assay

An HCV-1a ARMORED RNA (Ambion, Inc.; Austin, TX) that included HCV genomic sequences served as the template nucleic acid that was amplified in this procedure. ARMORED RNA® technology is used for producing ribonuclease-resistant RNA controls and standards by assembling specific RNA sequences and viral coat proteins into pseudo-viral particles. The template nucleic acid underwent specimen processing and target capture essentially according to the procedures disclosed in published International Patent Application No. PCT/US2000/18685, except that the template was captured using HCV-specific oligonucleotides rather than HIV-specific oligonucleotides. These procedures were conducted using 500 µl aliquots of stock samples having concentrations of HCV templates that ranged from 5 copies/ml up to 5 x 10⁷ copies/ml. An HCV pseudo target that contained sequences corresponding to amplification primer and target-capture oligonucleotide binding sites, together with a scrambled sequence corresponding to the probe binding site in accordance with U.S. Patent No. 6,294,338 was also included during the sample processing and target capture procedure. Neither the HCV pseudo target nor amplicons arising therefrom were capable of hybridizing with either Probe A or Probe B. Reagents for conducting TMA reactions, including amplification primers specific for HCV sequences, were combined with the HCV template/pseudo target mixture and pre-incubated for 10 minutes at 60°C to allow primer annealing. The mixtures were then cooled to 41.5°C for 10 minutes prior to addition of the MMLV reverse transcriptase and T7 RNA polymerase enzymes, essentially as described by Kacian et al., in U.S. Patent Nos. 5,399,491 and 5,554,516. TMA reactions were allowed to proceed for 60 minutes at 41.5°C. Amplified HCV target sequences were detected and quantified using AE-labeled probe reagents substantially as described previously (U.S. Patent No. 5,658,737 at column 25, lines 27-46; Nelson et al., Biochem. 35:8429 (1996)). More particularly, amplification products were hybridized with one of three probe reagents for 15 minutes at 60°C. In all cases the deoxy Probe A was labeled with a standard acridinium ester while the label on methoxy probe B had a methyl group at the 2 position of an acridinium ring (2-methyl-AE). In the first instance the reaction mixtures were hybridized with 4 pmols of deoxy Probe A that had been labeled to a specific activity of 1.5 x 10⁸ rlu/pmole. A parallel set of reaction mixtures were hybridized with 0.1 pmols of methoxy Probe B that had been labeled to a specific activity of 1.4 x 10⁸ rlu/pmole. Another parallel set of reaction mixtures was hybridized with the extended dynamic range probe reagent that included 4 pmols of labeled deoxy Probe A and 0.1 pmols of labeled methoxy Probe B. Following hybridization, label attached to unhybridized probe was inactivated and specifically hybridized probe quantified by luminometry essentially according to the procedure given by Arnold et al., in U.S. Patent No. 5,283,174, the disclosure of which is hereby incorporated by reference. After the selection step to inactivate any AE label associated with unhybridized probe, the samples were cooled to room temperature for 10-60 minutes, and total relative light units (RLU) determined using a LEADER HC luminometer (Gen-Probe Incorporated; San Diego, CA) and a 2 second read time, according to standard procedures that will be familiar to those having an ordinary level of skill in the art.

The results presented graphically in Figures 2A and 2B confirmed that the dynamic range of the hybridization and detection assay advantageously was extended by the use of two probes that hybridized the same analyte polynucleotide with different measurable binding interactions. As indicated in the figure, the hybridization assay conducted using only deoxy Probe A was capable of differentiating amplified target in the range spanning from a lower limit of between about 50-500 to an upper limit of about 5,000,000 input copies/ml (i.e., a 4-5 log range). Similarly, a hybridization assay conducted using only methoxy Probe B was capable of differentiating amplified target in the range spanning from about less than 5 to about 5,000 input copies/ml (i.e., a 3 log range). However, a combination of the two probes provided a useful dynamic range that spanned from about 5 to about 5,000,000 input copies/ml (i.e., a 6 log range). Notably, in this assay the probe that was used in the greater amount had a specific activity higher than the specific activity of the probe that was used in the lesser amount. This illustrates that it was not necessary to employ an inverse relationship between the amounts of the affinity-shifted probes and their specific activities when conducting extended dynamic range hybridization assays.

Inspection of the graphs shown in Figures 2A and 2B shows that deoxy Probe A when used alone was superior at quantifying amplicons at the higher concentration ranges that were tested, and that methoxy Probe B was superior at quantifying amplicons at the lower concentration ranges that were tested. For example, whereas the composite results obtained using the two probes in combination had a useful lower detection limit of 5 copies/ml, methoxy Probe B when used alone had the potential for quantifying fewer than 5 copies/ml. Thus, independently monitoring hybridization signals from multiple probes that hybridize the same analyte polynucleotide with different measurable binding interactions, for example using a microarray format or different labels producing signals that can be distinguished, can extend the dynamic range of the hybridization assay even further.

In an alternative procedure, the products of nucleic acid amplification reactions were hybridized with an affinity-shifted probe reagent that included a greater amount of the high affinity probe in combination with a lesser amount of the low affinity probe. In this procedure Probe B was labeled with standard AE to a specific activity of 1.25 x 10⁸, and Probe A was labeled with 2-methyl-AE to a specific activity of 1.85 x 10⁸. The two probes were used separately or combined to produce affinity-shifted probe reagents for hybridizing HCV amplicons that had been synthesized essentially as described above. Each hybridization reaction included either 0.6 pmoles of Probe B, or 0.3 pmoles of Probe A, or 0.6 pmoles of Probe B and 0.3 pmoles of Probe A. As before, when compared with Probe A, Probe B exhibited a higher affinity for the analyte amplicon. Results from this procedure again showed that the dynamic range of the hybridization assay had been extended by using a pair of affinity-shifted probes.

More specifically, the results presented graphically in Figures 3A and 3B again indicated that the dynamic range of the hybridization and detection assay advantageously had been extended by the use of two probes that hybridized the same analyte polynucleotide with different measurable binding interactions. As indicated in the figures, the hybridization assay conducted using only deoxy Probe A was capable of differentiating amplified target in the range spanning from a lower limit of about 50 to an upper limit of about 5,000,000 input copies/ml (i.e., a 5 log range). Similarly, and ignoring one outlying data point at 50 input copies/ml, a hybridization assay conducted using only methoxy Probe B was capable of differentiating amplified target in the range spanning from about less than 5 to between about 5,000-50,000 input copies/ml (i.e., about a 3-4 log range). However, a combination of the two probes provided a useful dynamic range that spanned from about 5 to about 5,000,000 input copies/ml (i.e., a 6 log range). Notably, in this assay the probe that was used in the greater amount had a specific activity lower than the specific activity of the probe that was used in the lesser amount. These results surprisingly demonstrated that extended dynamic range hybridization assays can be carried out using an amount of a higher affinity probe that is greater than or equal to the amount of a lower affinity probe. Stated differently, the amount of the probe used for quantifying higher amounts of an analyte can be lower than the amount of the probe used for quantifying the lower amounts of the same analyte.

In accordance with the foregoing demonstration, certain preferred assays use two hybridization probes having different affinities for an analyte polynucleotide, regardless of whether the analyte sequences hybridized by the probes are overlapping or non-overlapping, under conditions wherein the amount of the first probe is greater than or equal to the amount of the second probe, and the specific activity of the first probe is greater than or equal to the specific activity of the second probe.

The following several Examples describe procedures involving detection of HIV-1 amplicons. The probes used in the procedure were molecular beacons having overall lengths of from 21-26 nucleotides. These probes included 12-17 contiguous bases from the sequence GGGGUACAGUGCAGGGG (SEQ ID NO:16), and so were complementary to the HIV-1 amplicon. All probes were immobilized to solid supports by linkage through the target-complementary loop regions of the molecular beacons.

Example 3 describes methods used to demonstrate that a collection of probes that hybridized the same analyte polynucleotide species with different affinities could be used to quantify the analyte. In this procedure, a collection of molecular beacons that had been separately immobilized to different wells of a microtiter plate were contacted with known amounts of purified analyte polynucleotide under conditions of target excess.

### Example 3

### Molecular Beacons Having Different Affinities for a Single Target Polynucleotide Exhibit Differential Binding at Different Target Concentrations

Molecular beacon probes having the sequences of SEQ ID NOs:4-8 were independently synthesized by solid-phase phosphite triester chemistry using 3' DABCYL-linked controlled pore glass and 5' fluorescein-labeled phosphoramidite on a Perkin-Elmer (Foster City, CA) EXPEDITE model 8909 automated synthesizer. All of the molecular beacons were constructed using 2'-methoxy nucleotide analogs. Biotin moieties were introduced into the loop portions of the molecular beacon sequences (as indicated in Figure 5) using biotin phosphoramidite purchased from Glen Research Corporation (Sterling, VA). Following synthesis, the probes were deprotected and cleaved from the solid support matrix by treatment with concentrated ammonium hydroxide (30%) for two hours at 60°C. Next, the probes were purified using polyacrylamide gel electrophoresis followed by HPLC using standard procedures that will be familiar to those having an ordinary level of skill in the art.

The individual wells of a streptavidin-coated 96-well plastic microtiter plate (Laboratory Systems/Roche Diagnostics Corp.) were separately contacted with 20 pmoles of one of the 5 probes in 100 µl of binding buffer (100 mM Tris-HCl (pH 8.0), 0.1 M NaCl, 0.2 mM EDTA and 0.2% lithium lauryl sulfate) for a period of 1 hour at room temperature. At the conclusion of this immobilization step, each well containing an immobilized probe was washed 3 times with 100 µl of binding buffer. Each well was then contacted with a 100 µl aliquot containing 200 pmols of an RNA target having the sequence 5' UAUUCUUUCCCCUGCACUGUACCCCCCAAU 3' (SEQ ID NO:9) dissolved in binding buffer. This RNA target served as a model analyte polynucleotide in these procedures. The plate was incubated at room temperature for 1 hour to allow hybridization of the model analyte polynucleotide and the immobilized probes. Fluorescence emission at 525 nm was measured (in relative fluorescence units, or RFU) for each well following excitation at a wavelength of 495 nm. Quenching ratios calculated from these measurements are presented in Table 1. It is to be noted that the first column in the table particularly identifies the sequence of the molecular beacon probe and the length of the target-complementary loop sequence. Columns in Table 1 labeled "closed" and "open" refer to the expected configurations of the molecular beacon probes in the absence and presence of a complementary analyte polynucleotide, respectively.

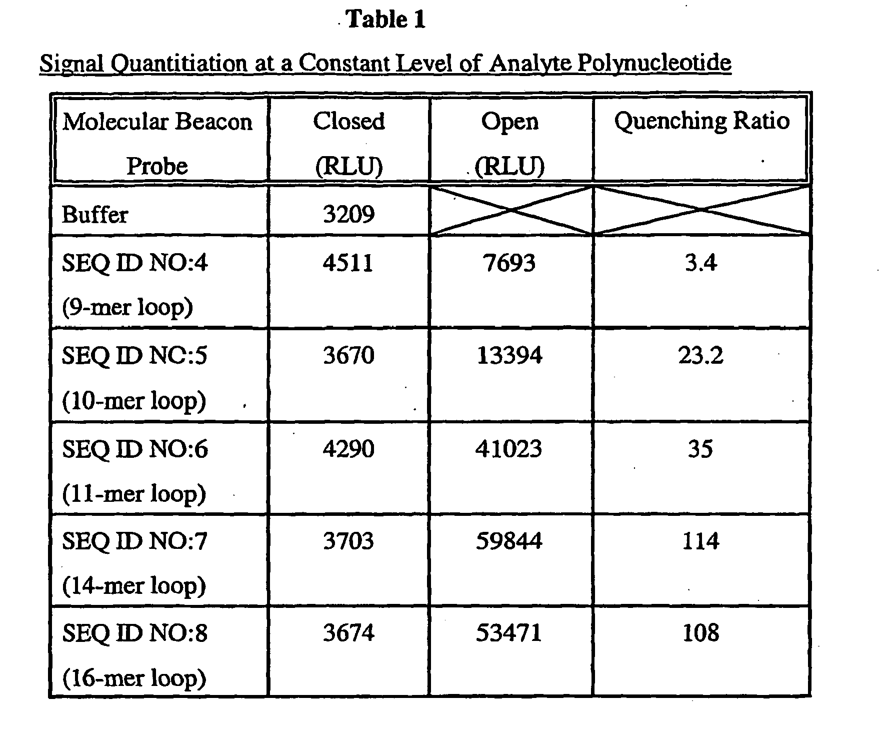

The results presented in Table 1 showed that the quenching ratio for each molecular beacon probe that hybridized the analyte polynucleotide was related to the length of the target-complementary loop sequence. Interestingly, except for the results obtained using the molecular beacon having the sequence of SEQ ID NO:8, the remaining data points in Table 1 substantially conformed to a linear relationship between the length of the target-complementary loop and the quenching ratio. More particularly, there was a nearly linear relationship between the length of the loop sequence and the quenching ratios determined for molecular beacons having lengths of 9, 10, 11 and 14 nucleotides. Only the result obtained using the molecular beacon having a target-complementary loop 16 nucleotides in length did not conform with this trend. Secondary structure in the loop portion of this molecular beacon (illustrated in Figure 5), but not in any of the other molecular beacons, was predicted by computer analysis using software based on methods disclosed by Mathews et al., in *J*. *Mol. Biol.* **288**:911 (1999). This finding demonstrates how differences in the sequences and/or secondary structures of the probes can affect the parameters for hybridization between a probe and an analyte polynucleotide.

The foregoing results established that the magnitudes of fluorescent signals emitted from immobilized self-reporting probes were related to the lengths of the target-complementary sequence, and so to the affinity of each probe for its target polynucleotide.

Having shown that different probes hybridized the same target polynucleotide and exhibited differential signaling at a single level of analyte polynucleotide, the magnitude of signaling from each species of molecular beacon across a range of target polynucleotide levels was next investigated.

Example 4 describes methods used to establish that a plurality of immobilized probe species that differentially interacted with the same target polynucleotide exhibited differential signaling over a broad range of target polynucleotide levels. A time course protocol was used to assess possible effects of incubation time on quantitative aspects of the hybridization procedure.

### Example 4

### Quantifying Analyte Polynucleotide Present in a Test Sample

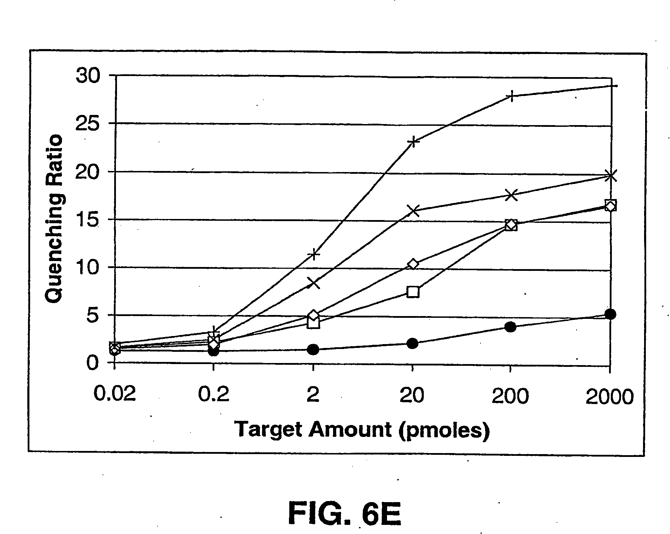

A microtiter plate having molecular beacon probes uniformly immobilized to the flat, inner surfaces of the wells was prepared essentially as described under Example 3. More specifically, 35 wells in the microtiter plate were divided into 5 sets of 7 wells per set. Each set of wells received one of the molecular beacons shown in Figure 5, using the procedures described in Example 3. After washing to remove probe that had not been immobilized, each well in the microtiter plate was contacted with a 100 µl aliquot containing either binding buffer alone or 0.02, 0.2, 2, 20, 200 and 2,000 pmols of the model analyte polynucleotide having the sequence of SEQ ID NO:9 dissolved in buffer. Trials conducted using known quantities of the analyte polynucleotide represented "standards" that provided a basis for comparison of results obtained using a test sample. In addition to these standard trials, one well for each of the different molecular beacon probes was contacted with a 100 µl aliquot representing a test sample. The plate was incubated at room temperature for periods of 1, 2, 3, 5 and 7 hours to allow hybridization of the analyte polynucleotide and the immobilized probe. Fluorescence measurements were made at each of these time points. Quenching ratios calculated from the fluorescence measurements, which indicated the extent of hybridization between molecular beacon probes and the analyte polynucleotide, are graphically presented in Figure 6 for all standard trials. Table 2 presents numerical results for all of the standards and the single test sample at the 1 hour time point. Molecular beacon probes listed in the table are identified by sequence identifiers with the lengths of the target-complementary loop sequences being indicated parenthetically.

**Table 2 Quenching Ratios Measured at 1 Hour**

| Amount of Standard (pmol) | Molecular Beacon Probe | | | | |
|---|---|---|---|---|---|
| | SEQ ID NO:4 (9-mer) | SEQ ID NO:5 (10-mer) | SEQ ID NO:6 (11-mer) | SEQ ID NO:7 (14-mer) | SEQ ID NO:8 (16-mer) |
| 0.02 | 1.18 | 1.3 | 1.19 | 1.55 | 1.35 |
| 0.2 | 1.125 | 1.49 | 1.46 | 2.14 | 1.89 |
| 2 | 1.21 | 2.04 | 2.41 | 5.49 | 4.79 |
| 20 | 1.45 | 2.85 | 5.4 | 12.7 | 11.19 |
| 200 | 2.22 | 5.4 | 9.6 | 20 | 15.21 |
| 2,000 | 3.13 | 6.6 | 11.27 | 22.4 | 17.35 |
| | | | | | |
| Unknown | 1.21 | 2.36 | 3.96 | 9.64 | 11.1 |

Results from these procedures confirmed that a plurality of immobilized molecular beacons which differed from each other in their target-complementary sequences rapidly gave fluorescent signals that were quantitatively related to the amount of target polynucleotide present in the microtiter well. More particularly, the sigmoid curves in each of the graphs shown in Figure 6 differ from each other at virtually all levels of target polynucleotide that were tested. Moreover, comparison of the graphs shown in Figure 6 indicated that substantially all of the fluorescent signal that would be produced by the immobilized molecular beacon was produced within the first hour of incubation at the highest level of analyte polynucleotide that was tested. For example, nearly 80% of the maximum fluorescent signal measured for the molecular beacon probe having the sequence of SEQ ID NO:7 at the 7 hour time point was already present after only one hour of incubation. Trials conducted using lesser amounts of standard polynucleotide showed increases in the hybridization signal between the 1 and 7 hour time points. Clearly, results from the 1 hour incubation were sufficient to permit quantitation of the analyte polynucleotide in the test sample. This shows that quantitation of an analyte polynucleotide using a collection of probes and the methods disclosed herein advantageously was very rapid

In addition to the convenience of rapid processing time, the use of multiple probes also enabled accurate measurement of the amount of analyte polynucleotide in the test sample. When numerical results obtained for the quenching ratios of the "unknown" trial presented in Table 2 were located on a first axis for each of the curves shown in Figure 6A, the corresponding target amount determined by consulting the second axis and then averaged, the result indicated that the test sample contained 9.6 pmoles of analyte polynucleotide. In fact, the model test sample contained 10 pmoles of analyte polynucleotide. This close agreement between the amount of analyte polynucleotide measured using the method described above, and the actual amount of analyte polynucleotide present in the test sample confirmed the utility of the quantitative probe array. Significantly, it was not necessary to ensure conditions of probe excess to achieve differential signaling in this quantitative system. Indeed, absolute amounts of analyte polynucleotide present in a test sample were accurately determined when the amount of analyte polynucleotide far exceeded the amount of probe.

The foregoing results confirmed that fluorescence signals from a plurality of different probes that hybridized the same analyte polynucleotide could be used for accurately determining the absolute amount of analyte polynucleotide in a test sample. These results were obtained using molecular beacons separately immobilized in different wells of a microtiter plate. Thus, the probes used in the procedures described in Examples 3 and 4 were spatially separated and were not in fluid communication with each other. The following Example describes procedures wherein molecular beacon probes were immobilized in the same well of a microtiter plate in a low density array format. The results obtained in this procedure showed how a plurality of different probes that hybridized the same analyte polynucleotide could be adapted to an array format. Moreover, the following Examples describe how quantitative probe arrays can be used in connection with nucleic acid amplification reactions to yield differential hybridization signals at the conclusion of amplification reactions conducted using different starting levels of template.

The procedures described herein can easily be used to quantify pre-amplification amounts of an analyte polynucleotide present in a test sample by interpreting post-amplification results obtained using the invented quantitative probe arrays. If a plurality of control amplification reactions are initiated using known numbers of molecules of an analyte polynucleotide standard, and if a test reaction is initiated using an unknown number of analyte polynucleotide molecules, then it becomes possible after measuring post-amplification hybridization signals in each reaction to determine the number of analyte polynucleotide molecules present in the test sample. The relationship between the number of molecules of analyte polynucleotide standard input into an amplification reactions and the amplicon-specific signal strength measured during or after the conclusion of the amplification reaction is most conveniently represented graphically. Determining the number of analyte polynucleotide molecules present in a test sample is simply a matter of determining from one or more standard control curves the number of analyte polynucleotide molecules corresponding to a measured analyte amplicon signal strength. This illustrates how analyte polynucleotide standards can be used in polynucleotide amplification reactions to quantify pre-amplification amounts of analyte polynucleotide contained in test samples. These same procedures are easily adapted for use in connection with quantitative probe arrays.

The following Example describes the use of three molecular beacon probes which differ from each other either by a single nucleotide in the length of the target-complementary loop sequence (i.e., comparing SEQ ID NO:10 and SEQ ID NO:12), or in the sequence of the arm portions that participate in stem formation (i.e., comparing SEQ ID NO:10 and SEQ ID NO:11). As shown below, changing either of these variables was sufficient to produce molecular beacons having differential interaction with a complementary analyte polynucleotide. Thus, collections of molecular beacons having different stem structures or different target-complementary loop sequences may be used for quantifying analyte polynucleotides in accordance with the present invention.

Example 5 describes the methods that were used to demonstrate detection and quantitation of a polynucleotide amplicon. In this instance the nucleic acid amplification reaction was carried out in the presence of a quantitative probe array.

### Example 5

### Detection and Quantitation of a Nucleic Acid Amplicon Using Quantitative Molecular Beacon Arrays

Three molecular beacon probes were prepared essentially as described in Example 3. The three probes were: WT014TEG having the sequence
CCGAGGGUACAGUGCAGGGCUCGG (SEQ ID NO:10), WT014C having the sequence GCGUGGGUACAGUGCAGGGCACGC (SEQ ID NO:11), and WT015 having the sequence CCGAGGGGUACAGUGCAGGGUUCGG (SEQ ID NO:12). All three probes were synthesized using 2'-methoxy nucleotide analogs and were biotinylated between positions 13 and 14 (for SEQ ID NO:10), between positions 12 and 13 (for SEQ ID NO:11), or between nucleotide positions 13 and 14 (for SEQ ID NO:12), also according to the methods described under Example 3. Probes were chemically coupled with Cy5 fluorophore labels (Pharmacia Corp.; Peapack, NJ) at their 5' ends, and BLACK HOLE QUENCHER™ moieties (Biosearch Technologies; Novato, CA) at their 3' ends. The molecular beacon probes were immobilized to streptavidin-coated 96-well plastic microtiter plates (Roche Diagnostics Corp.; Indianapolis, IN) as separately arrayed spots in a single 12-spot array using a BIOCHIP ARRAYER™ from Packard Bioscience Co. (Meriden, CT). Each spot in the array represented approximately 0.4 fmols of a single probe species. After spotting, the arrayed wells were washed twice with TENT buffer (50 mM Tris-HCl (pH7.5), 0.3 M NaCl, 0.1 M EDTA, 0.2% of the non-ionic wetting agent TWEEN-20 (a registered trademark of ICI Americas, Inc.)). This completed preparation of the probe array.

Side-by-side TMA reactions were carried out in the arrayed microtiter wells using 0, 300, 3000 or 30,000 copies of an HIV-1 RNA template polynucleotide that included the sequence of the *pol* gene. Amplification primers used in the procedure have been described by Bee et al., in published International Patent Application No. PCT/US00/18685. Methods used to carry out the amplification reactions were essentially as described by Kacian et al., in U.S. Patent No. 5,399,491. Briefly, 75 µl aliquots of a buffered solution containing primers, NTP and dNTP reactants were added to each well of the arrayed microtiter plate. Each well additionally received a 75 µl overlay of inert oil to control evaporation. The HIV-1 template polynucleotide was then added to the wells at levels ranging from 0-30,000 copies in volumes of from 1-1.5 µl. The plate was incubated at 60°C for 30 minutes, and then at 42°C for 15 minutes. Each well received a 25 µl aliquot of an enzyme reagent mix that included MMLV reverse transcriptase and T7 RNA polymerase to complete the reaction mixture. Wells of the plate were sealed by covering with a durable adhesive film, and then incubated at 42°C for 1 hour in a temperature-controlled shaker. At the conclusion of the reaction, the microtiter plate was brought to room temperature and scanned using a TYPHOON 8600 imager (Molecular Dynamics; Piscataway, NJ) to quantify fluorescent signals emitted from each spot in the array. The plate was then incubated at 65°C with shaking for 20 minutes, cooled to room temperature (about 23°C) for 20 minutes, and scanned again. This last procedure is referred to herein as a "heat/cool step." Averaged quantitative results from the scanning procedures prior to the heat/cool step are presented in Table 3. Averaged quantitative results from the scanning procedures following the heat/cool step are presented in Table 4. In all cases, quenching ratios were calculated by dividing the signal from the specified target copy level by the signal measured at the 0 target copy level.

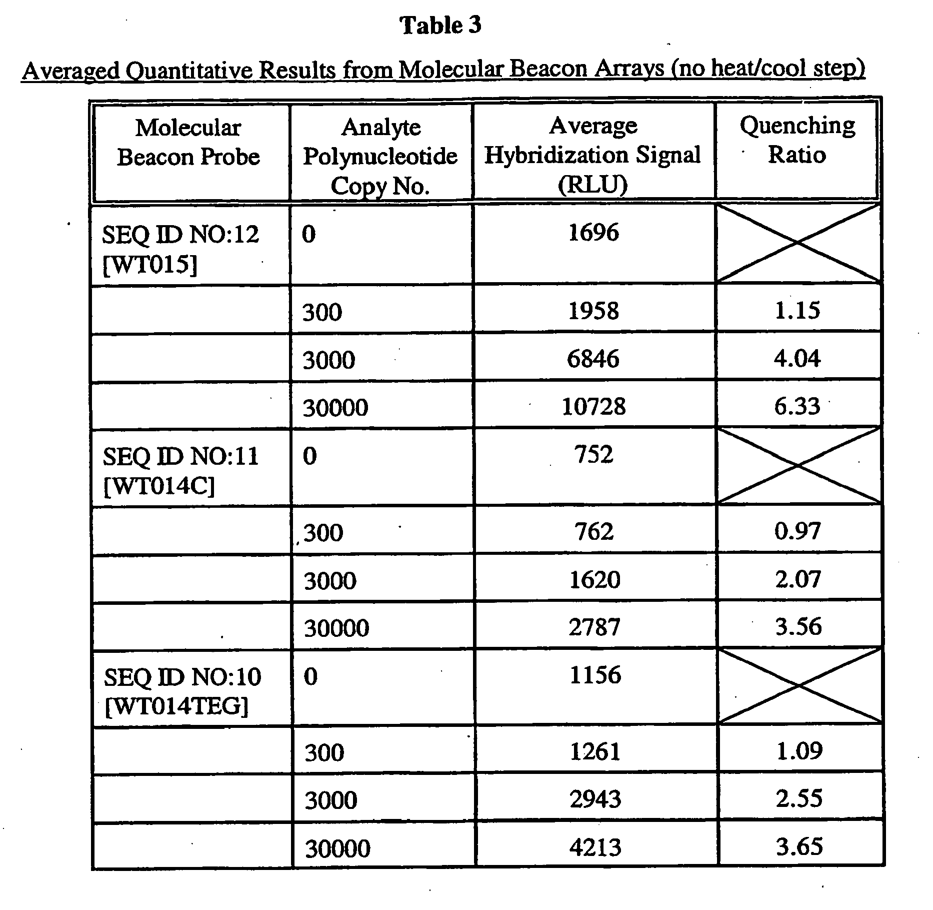

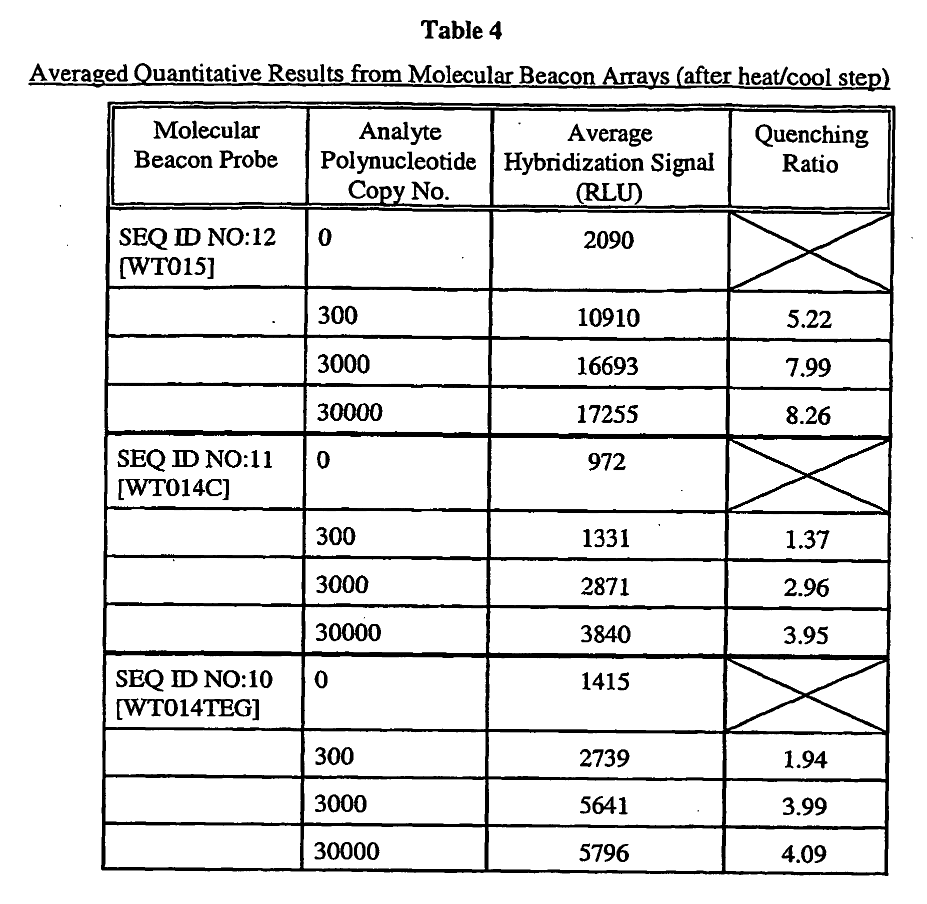

The results of these procedures confirmed that each of the three different probes yielded quantitatively different signals at each level of input template. It was unnecessary to maintain the immobilized probes separate from each other to achieve differential signaling at the conclusion of the amplification reaction, even across a broad range of input template levels. The results further confirmed that pairs of molecular beacons differing in either their target-complementary loop sequences or in the sequences of their stem regions could be used to achieve differential signaling that would be useful in the context of quantitative probe arrays. Significantly, amplification reactions could be conducted in the presence of the arrayed probes without evidence for inhibition. Finally, comparison of the results in Table 3 with the corresponding entries in Table 4 indicated that an optional heat/cool step at the conclusion of the amplification reaction advantageously increased the quenching ratios for all of the trials. In aggregate, these results indicated that amplification reactions could be conducted in the presence of arrayed self-reporting probes, and that signals generated by the self-reporting probes were useful for quantifying starting levels of analyte polynucleotide.

Example 6 describes methods that were used for preparing a quantitative probe array that included two different probe species. The two molecular beacons used in the exemplary arrays described below differed from each other by only three nucleotides in their target-complementary loop regions. Despite this seemingly small difference, the array showed differential signal production for TMA reactions conducted using input template levels extending over a range of at least 1000 fold.

### Example 6

### Quantitative Probe Array Having Two Probe Species

Two molecular beacon probes were prepared essentially as described in Example 3. The two probes were: WT016 having the sequence
CCGAGGGGUACAGUGCAGGGGCUCGG (SEQ ID NO:13), and WT013 having the sequence CCGAGGGUACAGUGCAGGCUCGG (SEQ ID NO:14). The molecular beacons were labeled with fluorophore and quencher moieties as described in Example 5. The probes were synthesized using 2'-methoxy nucleotide analogs and incorporated biotin residues joined through a non-nucleotide linker positioned between nucleotides 13 and 14 (for SEQ ID NO:13), or between nucleotides 12 and 13 (for SEQ ID NO:14). The probes were spotted onto the bottoms of streptavidin-coated plastic microtiter plates in an array format, and amplification reactions were carried out in the arrayed wells according to the method described in Example 5. At the conclusion of the amplification reactions the plate was exposed to an optional heat/cool step and then scanned to quantify the extent of hybridization. Quenching ratios for results at each level of input template polynucleotide were calculated. The averaged results of 5 replicates (for 0, 30, 300, 3000 template copies) or of 4 replicates (for 30,000 template copies) are presented in Table 5. Notably, the final column in the table shows the composite value resulting from the addition of quenching ratios measured for different probes at each level of input template. The values shown in the table are presented graphically in Figure 7.

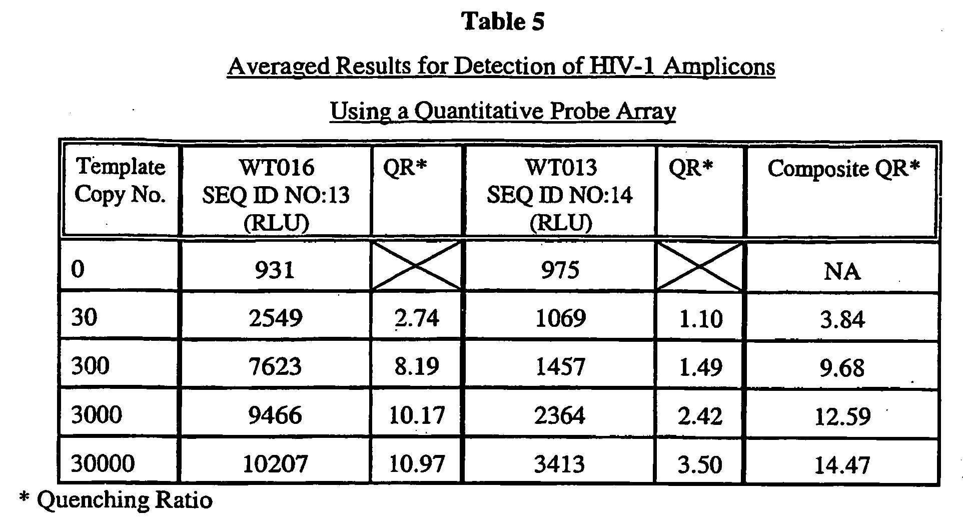

The results presented in Table 5 and in Figure 7 showed how amplification reactions using known amounts of analyte polynucleotide templates, and conducted in the presence of arrayed probes, yielded standard curves that related post-amplification hybridization signals and pre-amplification amounts of analyte polynucleotide. Indeed, the data presented in Figure 7 could serve as a standard control for comparison with results obtained in an amplification reaction conducted using an unknown input template copy number. Significantly, the standard curves generated using immobilized probe arrays in this procedure confirmed that signal strength was dependent on probe structure, and that two different probes gave different hybridization profiles, as expected.

The results from these procedures further illustrated how a composite curve generated by the superposition of hybridization signals measured for different probes in the array lead to an extended dynamic range. This composite curve shown in Figure 7 represents the magnitude of a hybridization signal that would be expected if both of the probes that had been separately arrayed were instead combined at a single spot in the array, and then subjected to the same amplification reaction. The composite curve advantageously increased over a greater range of template inputs than the rate of increase for curves representing either of the probes when used alone. This illustrates how a plurality of self-reporting hybridization probes may be used to obtain quantitative information over an extended dynamic range using an assay conducted in an array format.

Example 7 illustrates how the above-described quantitative probe array can be used to determine the amount of analyte polynucleotide present in a test sample. In this instance the standard curves of the preceding Example are used for interpreting quantitative results obtained in an assay conducted using a test sample containing an unknown number of HIV-1 analyte polynucleotides.

### Example 7

### Quantifying Analyte Polynucleotide by Amplification in the Presence of a Quantitative Probe Array

A test sample containing an unknown amount of HIV-1 polynucleotide is first obtained. A microtiter plate which includes arrayed molecular beacon probes is prepared as described in Example 6. Amplification reactions are prepared in separately arrayed wells of the microtiter plate using 0, 300, 3000 or 30000 copies of HIV-1 RNA standard dispensed in a volume of 1-1.5 µl, also as described in Example 6. One arrayed well in the microtiter plate is prepared using 1-1.5 µl of the test sample. Following addition of the enzyme reagent mix containing MMLV reverse transcriptase and T7 RNA polymerase, the microtiter plate is sealed and then incubated at 42°C for 1 hour in a temperature-controlled shaker. At the conclusion of the reaction, the microtiter plate is subjected to an optional heat/cool step and then scanned using a TYPHOON 8600 imager to quantify fluorescent signals emitted from each spot in the array. Signal-to-noise ratios are calculated for each trial using data obtained from the imager.

Results from the scanning procedure confirm that amplification reactions have taken place in each of the wells. Trials conducted using known amounts of HIV-1 RNA standard are plotted on a graph, and yield standard control curves exactly as shown in Figure 7. The trial conducted using the test sample gives quenching ratios of about 9.5 for the WT016 molecular beacon, and about 2.0 for the WT013 molecular beacon. These values are located on the vertical axis of the graph in Figure 7, and the corresponding number of copies of HIV-1 polynucleotide present in the sample determined from the horizontal axis of the graph. The test sample contains about 1000 copies of HIV-1 analyte polynucleotide. This illustrates how quantitative probe arrays can be used in connection with polynucleotide amplification reactions to determine the amount of analyte polynucleotide present in a test sample.

In another version of the procedure, the standard control curves are highly reproducible and it is unnecessary to perform amplification reactions using standards. Instead, the data representing the standard control curves is stored in an electronic memory device. An amplification reaction is conducted using the test sample in accordance with the procedures described above. Results obtained using the test sample give quenching ratios of about 9.5 for the WT016 molecular beacon, and about 2.0 for the WT013 molecular beacon. These values are input into an entry terminal and compared electronically with the standard curve stored in the memory device. An electronically generated output indicates the test sample contains 1000 copies of HIV-1 analyte polynucleotide. This illustrates an embodiment of the invention wherein it is unnecessary to conduct amplification reactions using standard controls.

An alternative method for quantifying polynucleotides that relies only on comparison of the calculated ratios of signals emitted from different molecular beacons can unambiguously make such determinations without particular knowledge about absolute signal values. This alternative method employs more than two different species of molecular beacon having binding specificity for the same polynucleotide, and relies on comparisons of a plurality of signal ratios. This method is illustrated below.

Example 8 describes methods that were used to produce control curves that were useful for unambiguously determining the amount of template polynucleotide is a sample prior to a nucleic acid amplification reaction. More specifically, the following procedures illustrate how results obtained using three different molecular beacons can be used for unambiguously determining starting levels of an analyte polynucleotide.

### Example 8

### Quantitative Probe Arrays Having More than Two Species of Molecular Beacon

The WT015dCG molecular beacon having the sequence CCGAGGGGUACAGUGCAGGGCUCGG (SEQ ID NO:15) was disposed along with the aforementioned WT016 and WT013 molecular beacons as separate spots in an array format at the bottoms of several wells of a streptavidin-coated plastic 96-well microtiter plate. The sequence of the WT015dCG molecular beacon differs from the sequence as the WT015 molecular beacon by a single nucleotide, so that a GU basepair in the stem of the WT015 molecular beacon was replaced by a GC basepair. Additionally, the WT015dCG molecular beacon was synthesized using 2'-methoxy nucleotide analogs at all positions except for positions 5 and 21, which were occupied by deoxyribonucleotides. A biotin moiety was linked to the probe between positions 13 and 14 using procedures described above. Arrayed wells were washed as described above. TMA reactions were then conducted in the arrayed wells, also as described above, using 30-30,000 copies of the HIV-1 template polynucleotide. Quenching ratios were calculated for each trial. These values were then used to calculate a "ratio of ratios" for the different combinations of molecular beacon probes at a particular level of input template. The summarized results presented in Table 6 show the signal ratios for the combinations of WT016/WT013, WT016/WT015dCG, and WT015dCG/WT013. The WT016/WT013 and WT016/WT015dCG ratio data are presented graphically in Figure 8. Since the WT015dCG/WT013 ratio is determinable from the other ratios listed in Table 6, those results are omitted from the figure.

**Table 6 Calculated Signal Ratios for Three Molecular Beacons**

| Input Template Copy No. | WT016/WT013 | WT016/WT015dCG | WT015dCG/WT013 |
|---|---|---|---|
| 0 | 1.18 | 1.47 | 0.81 |
| 30 | 2.56 | 1.71 | 1.49 |
| 300 | 4.73 | 1.76 | 2.69 |
| 3000 | 4.3 | 1.40 | 3.07 |
| 30000 | 3.7 | 1.29 | 2.86 |

The results from these procedures provided a simple and unambiguous method of determining the amount of template polynucleotide present in a sample prior to amplification by comparing signal ratios determined for a plurality of molecular beacons that bind the same target polynucleotide. Clearly, analysis of the signal ratios obtained using three molecular beacons uniquely identified the starting template copy number. This illustrates how comparisons among hybridization signals of as few as three different molecular beacons can uniquely determine the pre-amplification template copy number of an analyte polynucleotide.

It will be clear to those having an ordinary level of skill in the art that the data obtained in the fashion described in this Example can be used for determining the amount of analyte polynucleotide present in a test sample. For example, if a test sample run in parallel with the above-described standard controls had given WT016/WT013 and WT015dCG/WT013 signal ratios of about 4.1 and about 2.2, respectively, then consulting the graph shown in Figure 8 would unambiguously indicate that the sample contained about 100 copies of the template polynucleotide. This illustrates how immobilized arrays of self-reporting probes may be used to quantify low input template copy numbers.

## Claims

1. A method of quantifying the amount of an analyte polynucleotide present in a test sample over a range extending from a lower limit amount to an upper limit amount, comprising the steps of:
(a) providing a probe reagent comprising:
a first probe complementary to a first analyte sequence contained within the analyte polynucleotide, said first probe comprising a first oligonucleotide sequence, and
a second probe complementary to a second analyte sequence contained within the analyte polynucleotide, said second probe comprising a second oligonucleotide sequence,
wherein the first analyte sequence and the second analyte sequence are contiguous with each other and share at least one nucleotide position in common, and
wherein the first probe hybridizes to the analyte polynucleotide with a first affinity and the second probe hybridizes to the analyte polynucleotide with a second affinity, said first affinity and second affinity being different from each other;
(b) hybridizing said probe reagent to any of said analyte polynucleotide that may be present in said test sample, wherein the probe with the higher affinity hybridizes to the analyte polynucleotide in preference to the probe with lower affinity at any given level of analyte polynucleotide;
(c) measuring a signal that indicates the magnitude of hybrid duplex formation in step (b); and
(d) quantifying from the signal measured in step (c) the amount of said analyte polynucleotide present in said test sample,
thereby quantifying the amount of the analyte polynucleotide present in said test sample over a range of amounts that is greater than the amount of the analyte polynucleotide that can be detected by either the first probe or the second probe alone.

2. The method of Claim 1, wherein the measuring step comprises measuring optically.

3. The method of Claim 2, wherein the measuring step comprises performing luminometry.

4. The method of Claim 2, wherein the measuring step comprises measuring by fluorometry.

5. The method of Claim 1, wherein the quantifying step comprises comparing the signal measured in step (c) to a standard curve.

6. The method of Claim 1, wherein said first probe and said second probe are soluble probes.

7. The method of Claim 1, wherein said first probe and said second probe are immobilized probes.

8. The method of Claim 1, wherein either said first probe and said second probe are first and second soluble probes, or said first probe and said second probe are first and second immobilized probes.

9. The method of Claim 8, wherein the first oligonucleotide sequence comprises the complement of said first analyte sequence.

10. The method of Claim 8, wherein the first oligonucleotide sequence consists of the complement of said first analyte sequence.

11. The method of Claim 8, wherein said first oligonucleotide sequence and said second oligonucleotide sequence are identical to each other.

12. The method of Claim 11, wherein at least one of said first probe and said second probe comprises a nucleotide analog.

13. The method of Claim 8, wherein said first oligonucleotide sequence and said second oligonucleotide sequence are different from each other.

14. The method of Claim 13, wherein said first and said second probes are first and second self-reporting probes, each comprising a detectable label.

15. The method of Claim 14, wherein the detectable labels of the first and second self-reporting probes are identical detectable labels.

16. The method of Claim 8, wherein said first probe further comprises a first detectable label, and wherein said second probe further comprises a second detectable label.

17. The method of Claim 16, wherein said first detectable label and said second detectable label each comprise a chemiluminescent moiety.

18. The method of Claim 16, wherein said first detectable label and said second detectable label each comprise a fluorophore.

19. The method of Claim 17, wherein the chemiluminescent moiety of said first detectable label and the chemiluminescent moiety of the second detectable moiety are identical chemiluminescent moieties.

20. The method of Claim 19, wherein said identical chemiluminescent moieties comprise acridinium ester.

21. The method of Claim 6, wherein said first probe and said second probe are first and second linear probes.

22. The method of Claim 7, wherein said first probe and said second probe are first and second molecular beacon probes.

23. The method of Claim 8, wherein said first probe and said second probe are first and second self-reporting probes.

24. The method of Claim 23, wherein the first and second self-reporting probes are first and second molecular beacons.

25. The method of Claim 24, wherein the first and second molecular beacons each comprise a stem portion and a loop portion, and wherein said first and second molecular beacons differ from each other in the length of their respective stem portions.

26. The method of Claim 24, wherein the first and second molecular beacons each comprise a stem portion and a loop portion, and wherein said first and second molecular beacons differ from each other in the length of their respective loop portions.

27. The method of Claim 24, wherein at least one of said first and second molecular beacons comprises at least one nucleotide analog.

## Patentansprüche

1. Ein Verfahren zur Quantifizierung der Menge eines Polynukleotid-Analyts, das in einer Testprobe vorhanden ist, über einen Bereich, der sich von einer unteren Grenzwertmenge bis zu einer oberen Grenzwertmenge erstreckt, umfassend die Schritte:
(a) Bereitstellen eines Sondenreagens umfassend:
eine erste Sonde, die zu einer ersten Analytsequenz, die in dem Polynukleotid-Analyt enthalten ist, komplementär ist, wobei die erste Sonde eine erste Oligonukleotidsequenz umfasst, und
eine zweite Sonde, die zu einer zweiten Analytsequenz, die in dem Polynukleotid-Analyt enthalten ist, komplementär ist, wobei die zweite Sonde eine zweite Oligonukleotidsequenz umfasst,
wobei die erste Analytsequenz und die zweite Analytsequenz zusammenhängend sind und mindestens eine Nukleotidposition gemeinsam haben, und
wobei die erste Sonde an das Polynukleotid-Analyt mit einer ersten Affinität hybridisiert und die zweite Sonde an das Polynukleotid-Analyt mit einer zweiten Affinität hybridisiert, wobei die erste Affinität und die zweite Affinität unterschiedlich sind;
(b) Hybridisieren des Sondenreagens an jedes Polynukleotid-Analyt, das in der Testprobe vorhanden ist, wobei die Sonde mit der höheren Affinität gegenüber der Sonde mit niedrigerer Affinität bei jeder gegebenen Konzentration des Polynukleotid-Analyts bevorzugt an das Polynukleotid-Analyt hybridisiert;
(c) Messen eines Signals, das das Ausmaß der Hybrid-Duplex Bildung in Schritt (b) anzeigt; und
(d) Quantifizieren der Menge des Polynukleotid-Analyts, das in der Testprobe vorhanden ist, über das in Schritt (c) gemessene Signal,
wobei **dadurch** die Menge des Polynukleotid-Analyts, das in der Testprobe vorhanden ist, über einen Mengenbereich quantifiziert wird, der größer ist, als die Menge des Polynukleotid-Analyts, das durch entweder die erste oder zweite Sonde allein nachgewiesen werden kann

2. Das Verfahren nach Anspruch 1, wobei der Messschritt optisches Messen umfasst.

3. Das Verfahren nach Anspruch 2, wobei der Messschritt das Durchführen einer Luminometrie umfasst.

4. Das Verfahren nach Anspruch 2, wobei der Messschritt das Messen mittels Fluorometrie umfasst.

5. Das Verfahren nach Anspruch 1, wobei der Quantifizierungsschritt den Vergleich des in Schritt (c) gemessenen Signals mit einer Standardkurve umfasst.

6. Das Verfahren nach Anspruch 1, wobei die erste Sonde und die zweite Sonde lösliche Sonden sind.

7. Das Verfahren nach Anspruch 1, wobei die erste Sonde und die zweite Sonde immobilisierte Sonden sind.

8. Das Verfahren nach Anspruch 1, wobei entweder die erste Sonde und die zweite Sonde erste und zweite lösliche Sonden sind, oder die erste Sonde und die zweite Sonde erste und zweite immobilisierte Sonden sind.

9. Das Verfahren nach Anspruch 8, wobei die erste Oligonukleotidsequenz das Komplementär der ersten Analytsequenz umfasst.

10. Das Verfahren nach Anspruch 8, wobei die erste Oligonukleotidsequenz aus dem Komplementär der ersten Analytsequenz besteht.

11. Das Verfahren nach Anspruch 8, wobei die erste Oligonukleotidsequenz und die zweite Oligonukleotidsequenz identisch sind.

12. Das Verfahren nach Anspruch 11, wobei mindestens eine der ersten Sonde und der zweiten Sonde ein Nukleotidanalog umfasst.

13. Das Verfahren nach Anspruch 8, wobei die erste Oligonukleotidsequenz und die zweite Oligonukleotidsequenz unterschiedlich sind.

14. Das Verfahren nach Anspruch 13, wobei die erste und zweite Sonde erste und zweite selbst-berichtende Sonden sind, wobei jede einen nachweisbaren Marker umfasst.

15. Das Verfahren nach Anspruch 14, wobei die nachweisbaren Marker der ersten und zweiten selbst-berichtenden Sonden identische nachweisbare Marker sind.

16. Das Verfahren nach Anspruch 8, wobei die erste Sonde ferner einen ersten nachweisbaren Marker umfasst und wobei die zweite Sonde ferner einen zweiten nachweisbaren Marker umfasst.

17. Das Verfahren nach Anspruch 16, wobei der erste nachweisbare Marker und der zweite nachweisbare Marker jeweils einen chemolumineszenten Rest umfassen.

18. Das Verfahren nach Anspruch 16, wobei der erste nachweisbare Marker und der zweite nachweisbare Marker jeweils ein Fluorophor umfassen.

19. Das Verfahren nach Anspruch 17, wobei der chemolumineszente Rest des ersten nachweisbaren Markers und der chemolumineszente Rest des zweiten nachweisbaren Markers identische chemolumineszente Reste sind.

20. Das Verfahren nach Anspruch 19, wobei die identischen chemolumineszenten Reste Acridiniumester umfassen.

21. Das Verfahren nach Anspruch 6, wobei die erste Sonde und die zweite Sonde erste und zweite lineare Sonden sind.

22. Das Verfahren nach Anspruch 7, wobei die erste Sonde und die zweite Sonde erste und zweite molecular beacon-Sonden sind.

23. Das Verfahren nach Anspruch 8, wobei die erste Sonde und die zweite Sonde erste und zweite selbst-berichtende Sonden sind.

24. Das Verfahren nach Anspruch 23, wobei die erste und zweite selbst-berichtende Sonde erster und zweiter molecular beacon sind.

25. Das Verfahren nach Anspruch 24, wobei der erste und zweite molecular beacon jeweils einen Stammanteil und einen Schleifenanteil umfassen, und wobei der erste und zweite molecular beacon sich durch die Länge ihrer jeweiligen Stammanteile voneinander unterscheiden.

26. Das Verfahren nach Anspruch 24, wobei der erste und zweite molecular beacon jeweils einen Stammanteil und einen Schleifenanteil umfassen, und wobei der erste und zweite molecular beacon sich durch die Länge ihrer jeweiligen Schleifenanteile voneinander unterscheiden.

27. Das Verfahren nach Anspruch 24, wobei mindestens einer von dem ersten und zweiten molecular beacon mindestens ein Nukleotidanalog umfasst.

## Revendications

1. Procédé de quantification de la quantité d'un polynucléotide analyte présent dans un échantillon à tester sur une gamme s'étendant d'une quantité limite inférieure à une quantité limite supérieure, comprenant les étapes consistant à:
(a) fournir un réactif sonde comprenant :
une première sonde complémentaire d'une première séquence d'analyte contenue dans le polynucléotide analyte, ladite première sonde comprenant une première séquence oligonucléotidique, et
une seconde sonde complémentaire d'une seconde séquence d'analyte contenue dans le polynucléotide analyte, ladite seconde sonde comprenant une seconde séquence oligonucléotidique,
dans lequel la première séquence d'analyte et la seconde séquence d'analyte sont contiguës l'une à l'autre et partagent au moins une position de nucléotide en commun, et
dans lequel la première sonde s"hybride au polynucléotide analyte avec une première affinité et la seconde sonde s'hybride au polynucléotide analyte avec une seconde affinité, lesdites première affinité et seconde affinité étant différentes l'une de l'autre ;
(b) hybrider ledit réactif sonde à tout dit polynucléotide analyte qui peut être présent dans ledit échantillon à tester, dans lequel ladite sonde avec l'affinité plus élevée s'hybride au polynucléotide analyte de préférence à la sonde avec une affinité plus faible à tout niveau donné de polynucléotide analyte ;
(c) mesurer un signal qui indique l'amplitude de la formation de duplexe hybride dans l'étape (b) ; et
(d) quantifier d'après le signal mesuré dans l'étape (c) la quantité dudit polynucléotide analyte présent dans ledit échantillon à tester.
en quantifiant ainsi la quantité de polynucléotide analyte présent dans ledit échantillon à tester dans une gamme de quantités qui est plus importante que la quantité du polynucléotide analyte qui peut être détectée par la première sonde ou la seconde sonde seules.

2. Procédé de la revendication 1, dans lequel l'étape de mesure comprend une mesure optique.

3. Procédé de la revendication 2, dans lequel l'étape de mesure comprend la mise en oeuvre de la luminométrie.

4. Procédé de la revendication 2, dans lequel l'étape de mesure comprend une mesure par fluorométrie.

5. Procédé de la revendication 1, dans lequel l'étape de quantification comprend la comparaison du signal mesuré dans l'étape (c) à une courbe étalon.

6. Procédé de la revendication 1, dans lequel ladite première sonde et ladite seconde sonde sont des sondes solubles.

7. Procédé de la revendication 1, dans lequel ladite première sonde et ladite seconde sonde sont des sondes immobilisées.

8. Procédé de la revendication 1, dans lequel ladite première sonde et ladite seconde sonde sont des première et seconde sondes solubles, ou ladite première sonde et ladite seconde sonde sont des première et seconde sondes immobilisées.

9. Procédé de la revendication 8, dans lequel la première séquence oligonucléotidique comprend le complément de ladite première séquence d'analyte.

10. Procédé de la revendication 8, dans lequel la première séquence oligonucléotidique est constituée du complément de ladite première séquence d'analyte.

11. Procédé de la revendication 8, dans lequel ladite première séquence oligonucléotidique et ladite seconde séquence oligonucléotidique sont identiques l'une à l'autre.

12. Procédé de la revendication 11, dans lequel au moins une parmi ladite première sonde et ladite seconde sonde comprend un analogue de nucléotide.

13. Procédé de la revendication 8, dans lequel ladite première séquence oligonucléotidique et ladite seconde séquence oligonucléotidique sont différentes l'une à l'autre.

14. Procédé de la revendication 13, dans lequel ladite première sonde et ladite seconde sonde sont des première et seconde sondes auto-reporters, chacune comprenant un marqueur détectable.

15. Procédé de la revendication 14, dans lequel les marqueurs détectables de la première et de la seconde sondes auto-reporters sont des marqueurs détectables identiques.

16. Procédé de la revendication 8, dans lequel ladite première sonde comprend en outre un premier marqueur détectable, et dans lequel ladite seconde sonde comprend en outre un second marqueur détectable.

17. Procédé de la revendication 16, dans lequel ledit premier marqueur détectable et ledit second marqueur détectable comprennent chacun une partie chimioluminescente.

18. Procédé de la revendication 16, dans lequel ledit premier marqueur détectable et ledit second marqueur détectable comprennent chacun un fluorophore.

19. Procédé de la revendication 17, dans lequel la partie chimioluminescente dudit premier marqueur détectable et la partie chimioluminescente de ladite seconde partie détectable sont des parties chimioluminescentes identiques.

20. Procédé de la revendication 19, dans lequel lesdites parties chimioluminescentes identiques comprennent de l'ester d'acridinium.

21. Procédé de la revendication 6, dans lequel ladite première sonde et ladite seconde sonde sont des première et seconde sondes linéaires.

22. Procédé de la revendication 7, dans lequel ladite première sonde et ladite seconde sonde sont des première et seconde sondes balises moléculaires.

23. Procédé de la revendication 8, dans lequel ladite première sonde et ladite seconde sonde sont des première et seconde sondes auto-reporters.

24. Procédé de la revendication 23, dans lequel ladite première sonde et ladite seconde sonde auto-reporters sont des première et seconde balises moléculaires.

25. Procédé de la revendication 24, dans lequel la première et la seconde balises moléculaires comprennent chacune une portion de tige et une portion de boucle, et dans lequel lesdites première et seconde balises moléculaires diffèrent l'une de l'autre par la longueur de leurs portions de tige respectives.

26. Procédé de la revendication 24, dans lequel la première et la seconde balises moléculaires comprennent chacune une portion de tige et une portion de boucle, et dans lequel lesdites première et seconde balises moléculaires diffèrent l'une de l'autre par la longueur de leurs portions de boucle respectives.

27. Procédé de la revendication 24, dans lequel au moins une desdites première et seconde balises moléculaires comprennent au moins un analogue de nucléotide.
